# EUROPEAN PATENT SPECIFICATION

(11) **EP 4 593 691 B1**
(45) Date of publication and mention of the grant of the patent: **09.09.2026**
(21) Application number: 23793640.6
(22) Date of filing: 25.09.2023
(51) Int. Cl.: A61B 5/00, A61N 1/05, A61B 5/0215

(54) **TANDEM INTERLACE DELIVERY CATHETER FOR DELIVERING AN INTRACORPOREAL SENSOR**
TANDEM-ZWISCHENVERBINDUNGS-FREISETZUNGSKATHETER ZUR FREISETZUNG EINES INTRAKORPORALEN SENSORS
CATHÉTER D'ADMINISTRATION EN TANDEM ENTRELACÉ POUR L'ADMINISTRATION D'UN CAPTEUR INTRACORPOREL

(30) Priority: 30.09.2022 US 202263412003 P; 22.11.2022 US 202263427122 P; 22.09.2023 US 202318472520
(43) Date of publication of application: 06.08.2025
(73) Proprietor: TC1 LLC, St. Paul, MN 55117 (US)
(72) Inventor: HALL, Lindsay, St. Paul, MN 55117 (US); SETTLE, Jenna, St. Paul, MN 55117 (US); EIDENSCHINK, Tracee, St. Paul, MN 55117 (US)
(74) Representative: Barker Brettell Sweden AB
(86) International application number: PCT/US2023/074982
(87) International publication number: WO 2024/073322

(56) References cited:
- EP-B1- 2 833 966
- EP-B1- 3 307 212
- US-A1- 2003 114 735
- US-A1- 2020 146 562
- US-A1- 2021 290 165
- US-B2- 8 727 996

## Description

### BACKGROUND

Embodiments of the present disclosure generally relate to methods and devices to secure an intracorporeal device to a device delivery system and release the device at a desired location within a body.

Implantable (e.g., intracorporeal) medical sensors are currently available to monitor certain physiologic conditions, such as blood pressure. The size of the implantable medical sensor is limited due to target implant locations within the patient, such as within blood vessels. One example of an implantable medical sensor is a pulmonary arterial (PA) pressure sensor. In some cases, the sensors can be passive, utilizing an external device located outside of the patient body for supplying energy to power the generation and/or communication of the physiological data. In other cases, the sensors may have an onboard battery capable of limited functionality.

Currently, when delivering an implantable sensor within a vessel, the sensor is held in parallel with the delivery catheter, in other words, the sensor is stacked on top of the delivery catheter. The delivery catheter systems utilize catheters that have a plurality of openings or skives, such as eight skives, along an outer surface of the catheter. Using the skives, the sensor to be delivered is tethered to the catheter at a plurality of points in the stacked configuration. Therefore, the total profile that must fit within an introducer sheath, which in some cases can have a 12F introducer lumen, is the outer diameter of the catheter plus the height/width of the sensor. In many cases it is desirable to decrease the overall diameter of the sensor delivery system such that smaller introducer sheaths can be used and to facilitate the use with a wider variety of patients.

When removing the tether(s), the tether wire(s) must be pulled through the number of skives or openings, resulting in friction that can inhibit the pulling force. Further, due to the stacked configuration and tethering locations, when the sensor is released, catheter material can be located distal of the sensor and must still be removed which can increase difficulty of retraction.

A need remains for methods and devices that improve the delivery and release of the implantable device.

EP 2833966 B1 discloses a kit for intravascular implantation of an implantable medical device within a patient comprising an elongated outer sheath forming an inner lumen with a distal opening, the outer sheath sized to traverse a vasculature of the patient, and an elongated inner sheath with a stopper. The inner sheath further includes a tether configured to form a loop on a distal side of the stopper, the loop being configured to engage a looped element of the implantable medical device to couple the implantable medical device to the inner sheath. The stopper is slidable relative to the outer sheath. The tether is configured to release the looped element of the implantable medical device from the inner sheath by opening the tether loop when a portion of the stopper is located distally relative to the distal opening of the outer sheath.

EP 3307212 B1 discloses a delivery system including an outer catheter having a distal tip and an inner support member disposed within the outer catheter. The inner support member includes an anchor member adjacent a distal tip of the inner support member and a support portion axially inward of the anchor member. The support portion is configured for supporting an implantable device thereon. A diameter of the anchor member corresponds to a diameter of a portion of a vessel in which the anchor member is to be disposed. The anchor member is configured to be lodged in the portion of the vessel to locate an intended position of the anchor member and to prevent movement of the inner support member relative to the vessel during release of the implantable device.

US 2020/146562 A1 discloses an implant delivery system comprising an implant, a first sheath and a second sheath each extending from a proximal end of the implant delivery system. The first sheath is translatable relative to said second sheath and the implant is connected to an exterior surface of the first sheath. The first sheath and said second sheath are movable with respect to one another to deploy the implant to a target site in an anatomy. The delivery system is partially inserted into a blood vessel of a human body such that the proximal end remains external to the body and the distal end is internal to the body.

### SUMMARY

In accordance with embodiments herein an intracorporeal sensor delivery system comprises a delivery catheter comprising a lumen. The delivery catheter has a proximal end and a distal end. A guide wire lumen (GWL) is configured to receive a guide wire, and the GWL extends within the lumen of the catheter and protrudes beyond the distal end of the catheter. An intracorporeal sensor comprises a proximal end and a distal end. The proximal end of the sensor is positioned distal to the distal end of the catheter. A proximal coupling feature is coupled to the proximal end of the sensor. The proximal coupling feature is configured to removably couple the sensor to the delivery catheter. A distal coupling feature is coupled to the distal end of the sensor. The distal coupling feature is further removably coupled around an outer surface of the GWL at a position that is distally located with respect to the distal end of the sensor.

Optionally, the distal coupling feature comprises a loop that is interlaced around the outer surface of the GWL. In some cases, the loop comprises a first loop portion and a second loop portion. The first loop portion is attached to the sensor at a first attachment point. The second loop portion is attached to the sensor at a second attachment point. The first and second loop portions of the distal coupling feature are interlaced to form a first set of cross points along a first side of the GWL and a second set of cross points along a second side of the GWL. The first loop portion is outside of the second loop portion at the first set of cross points and the second loop portion is outside of the first loop portion at the second set of cross points.

Optionally, the loop comprises a first loop portion and a second loop portion. The first loop portion is attached to the sensor at a first attachment point. The second loop portion is attached to the sensor at a second attachment point. The first and second loop portions are interlaced to form a first set of cross points along a first side of the GWL and a second set of cross points along a second side of the GWL. The second loop portion is positioned between the first loop portion and the outer surface of the GWL at the first set of cross points and the first loop portion is positioned between the second loop portion and the outer surface of the GWL at the second set of cross points.

Optionally, the distal coupling feature comprises a loop that is twisted or wrapped around the outer surface of the GWL. Optionally, the proximal coupling feature comprises a loop that is twisted at least once around itself. Optionally, the GWL comprises a length, wherein a first portion of the length has a first stiffness, and a second portion of the length has a second stiffness that is different from the first stiffness. Optionally, the distal coupling feature comprises a loop that is wrapped around the outer surface of the GWL to allow, in response to the GWL being decoupled from the coupling feature, the loop to open laterally with no axial twisting, the opened loop configured to engage walls of a vessel to provide rotational stability of the sensor.

Optionally, the system further comprises a pressure sheath configured to removably cover a portion of the catheter and a portion of the proximal coupling feature. Optionally, the sensor is a pressure sensor.

Optionally, the catheter further comprises a second lumen extending parallel with respect to the lumen. Optionally, the catheter further comprises a skive into a lumen positioned proximal to the distal end of the catheter. The skive is configured to receive the proximal coupling feature, and the proximal coupling feature comprises a loop extending through the skive and between the GWL and the catheter.

Optionally, the catheter further comprises a skive positioned proximal to the distal end of the catheter. The skive is configured to receive the proximal coupling feature, and the proximal coupling feature comprises a loop interlaced around the outer surface of the GWL. The first and second loop portions are interlaced to form a first set of cross points along a first side of the GWL and a second set of cross points along a second side of the GWL. The second loop portion is positioned between the first loop portion and the outer surface of the GWL at the first set of cross points and the first loop portion is positioned between the second loop portion and the outer surface of the GWL at the second set of cross points.

Optionally, the distal coupling feature comprises a loop that is twisted or wrapped around the outer surface of the GWL. Optionally, the proximal coupling feature comprises a loop that is twisted at least once around itself. Optionally, the GWL comprises a length, wherein a first portion of the length has a first stiffness, and a second portion of the length has a second stiffness that is different from the first stiffness. Optionally, the distal coupling feature comprises a loop that is wrapped around the outer surface of the GWL to allow, in response to the GWL being decoupled from the coupling feature, the loop to open laterally with no axial twisting, the opened loop configured to engage walls of a vessel to provide rotational stability of the sensor.

Optionally, the system further comprises a pressure sheath configured to removably cover a portion of the catheter and a portion of the proximal coupling feature. Optionally, the sensor is a pressure sensor.

Optionally, the catheter further comprises a second lumen extending parallel with respect to the lumen. Optionally, the catheter further comprises a skive into a lumen positioned proximal to the distal end of the catheter. The skive is configured to receive the proximal coupling feature, and the proximal coupling feature comprises a loop extending through the skive and between the GWL and the catheter.

Optionally, the catheter further comprises a skive positioned proximal to the distal end of the catheter. The skive is configured to receive the proximal coupling feature, and the proximal coupling feature comprises a loop interlaced around the outer surface of the GWL.

In accordance with embodiments herein, an intracorporeal sensor delivery system comprises a first lumen configured to receive a GWL. The GWL is configured to extend beyond a distal end of the first lumen. The GWL is configured to removably receive a distal coupling feature coupled to an outer surface of the GWL at a position that is distally located with respect to the distal end of the lumen, the distal coupling feature interconnected with a sensor. A second lumen is configured to convey a release mechanism. The release mechanism is configured to removably couple with a proximal end of the sensor, wherein the first and second lumens are positioned in parallel and held together.

Optionally, the release mechanism is a floss or a threaded fastener. Optionally, the system includes a cutter mechanism extending within the second lumen, the cutter mechanism configured to sever the release mechanism. Optionally, the system further comprises a pressure lumen positioned in parallel and held together with the first and second lumens. Optionally, the release mechanism is a floss, wherein the release mechanism is configured to extend through a hole or a ring in the proximal end of the sensor.

In accordance with embodiments herein, a method for forming a tandem delivery system configured to deliver an intracorporeal sensor comprises positioning a sensor and catheter in tandem wherein a proximal end of the sensor is located distal with respect to the catheter, and a GWL extending through the catheter is configured to extend beyond a distal end of the sensor. The method includes removably coupling a distal coupling feature around an outer surface of the GWL that extends beyond the distal end of the sensor, and the distal coupling feature is coupled to the sensor. The method further includes removably coupling a proximal coupling feature to the catheter, and wherein the proximal coupling feature is coupled to the sensor.

Optionally, the proximal coupling feature extends between the outer surface of the GWL and an inner portion of the catheter. Optionally, the removably coupling of the proximal coupling feature to the catheter further comprises removably retaining the proximal coupling feature within a skive formed in an outer surface of the catheter, wherein the GWL spans the skive, and the method further comprises capturing the proximal coupling feature between the outer surface of the GWL and the skive.

Optionally, the method further comprising interlacing the distal coupling feature around an outer surface of the GWL.

Optionally, the distal coupling feature includes first and second loop portions that are interlaced together to form first cross points on a first side of the GWL and second cross points on a second side of the GWL. The method further comprises positioning the first loop portion between the second loop portion and the GWL to form the first cross points on the first side of the GWL, and positioning the second loop portion between the first loop portion and the GWL to form the second cross points on the second side of the GWL.

In accordance with embodiments herein, an intracorporeal sensor delivery system comprises a catheter, a guide wire lumen (GWL), and a distal coupling feature. The delivery catheter comprises a lumen and the catheter has a proximal end and a distal end. The GWL is configured to receive a guide wire, and the GWL extends within the lumen of the catheter and protrudes beyond the distal end of the catheter. The distal coupling feature is coupled to a distal end of a sensor and is further removably coupled to an outer surface of the GWL at a position that is distally located with respect to the distal end of the catheter.

Optionally, the distal coupling feature comprises a loop that is interlaced around the outer surface of the GWL. Optionally, the distal coupling feature comprises a loop that is removably captured by a removable floss that is tethered to the outer surface of the GWL with at least one bumper. Optionally, the system further comprises a proximal coupling feature configured to removably couple at a proximal end of the sensor, wherein the proximal coupling feature is one of a floss, a threaded fastener, a snare, paddles, or detents. Optionally, the catheter comprises a second lumen, the system further comprising a torque cable extending through the second lumen, the torque cable comprising a proximal coupling feature configured to removably couple to a proximal end of the sensor.

Optionally, the catheter comprises a pressure sheath configured to measure pressure. Optionally, the catheter comprises a second lumen, and the system further comprises a push rod extending through the second lumen, the push rod further comprising a cutting element.

Optionally, a sensor comprises a proximal end and the distal end. A proximal coupling feature is coupled to the proximal end of the sensor. The proximal coupling feature is configured to removably couple the sensor to the delivery catheter. A pressure sheath is configured to removably cover a portion of the catheter and a portion of the proximal coupling feature.

Optionally, a sensor comprises a proximal end and the distal end. A proximal coupling feature coupled to i) the catheter or ii) the GWL, wherein the distal coupling feature and the proximal coupling feature, in response to the sensor being positioned within a vessel and being removably coupled from the GWL or catheter, are configured to interface with walls of the vessel to secure the sensor within the vessel.

Optionally, an intracorporeal sensor delivery system comprises a delivery catheter, a guide wire lumen (GWL), and a proximal external sleeve. The sleeve will act as a lumen to extract pressure readings from the anatomy. The sleeve is located from the proximal end of the catheter and extends up to the distal end of the catheter. The sleeve may be shorter than the shaft of the catheter so the sleeve can be advanced or retracted after sensor release.

### BRIEF DESCRIPTION OF THE DRAWINGS

Figure 1A illustrates a sensor delivery system in accordance with embodiments herein.
Figure 1B illustrates a method for releasing the sensor within a vessel after the sensor is positioned at the desired location in accordance with embodiments herein.
Figures 2A and 2B illustrate delivery paths that can be used to position a delivery sheath, and thus the sensor delivery system, in place within a patient in accordance with embodiments herein.
Figure 3A illustrates a cross-sectional view of sensor delivery system and a cross-sectional view of a multi-lumen catheter in accordance with embodiments herein.
Figure 3B shows a variable stiffness GWL in accordance with embodiments herein.
Figure 3C shows a view of the skive in the multi-lumen catheter with the proximal loop captured between the guide wire lumen (GWL) and the skive of the catheter in accordance with embodiments herein.
Figure 3D shows a view of the sensor delivery system in accordance with embodiments herein.
Figure 3E illustrates a cross-sectional view of the sensor delivery system wherein the multi-lumen catheter includes two skives in accordance with embodiments herein.
Figures 3F, 3G, and 3H show cross-sectional views of the sensor and the multi-lumen catheter positioned in tandem and viewed in the proximal direction in accordance with embodiments herein.
Figure 3I illustrates an end cross-sectional view of the multi-lumen catheter having the GWL extending through a larger or second lumen in accordance with embodiments herein.
Figure 3J shows a cross-sectional view of a mono-lumen catheter that has an asymmetric bumper with an outer diameter that is larger than the smaller diameter catheter shaft in accordance with embodiments herein.
Figure 3K illustrates a sensor delivery system that utilizes a tri-lumen catheter to deliver the sensor within the body in accordance with embodiments herein.
Figure 3L shows a portion of the tri-lumen catheter wherein the GWL lumen, torque cable lumen, and pressure lumen are linearly aligned in accordance with embodiments herein.
Figure 3M shows an end view of the tri-lumen catheter having the GWL lumen, torque cable lumen, and pressure lumen arranged triangularly with respect to each other and encased in heat shrink in accordance with embodiments herein.
Figure 3N is another view of the tri-lumen catheter that shows the sensor in accordance with embodiments herein.
Figures 4A-4C illustrate top-down views of interlacing of the distal loop on the GWL in accordance with embodiments herein.
Figure 4D illustrates the sensor and distal loop secured to the sensor at attachment points in accordance with embodiments herein.
Figures 5A and 5B illustrate cross-sectional views of proximal loop interlacing in accordance with embodiments herein.
Figure 5C illustrates a cross-sectional view of the proximal loop retained by the GWL wherein portions of the proximal loop are twisted together or around each other in accordance with embodiments herein.
Figures 6Aand 6B illustrate a pressure sheath positioned over at least a portion of the catheter in accordance with embodiments herein.
Figure 6C illustrates translational components on the proximal end of the catheter that allow for translational control of the sensor delivery system.
Figure 6D illustrates a method for forming a tandem delivery system that can deliver an intracorporeal sensor in accordance with embodiments herein.
Figure 7A illustrates a cross-sectional view of the sensor and catheter positioned in tandem, wherein the distal loop is secured to the GWL with floss and rubber rings or bumpers in accordance with embodiments herein.
Figure 7B illustrates another view of the delivery system of Figure 7A in accordance with embodiments herein.
Figure 7C shows a cross-sectional view of the bumper encircling the GWL and Figure 7D includes the floss held between the bumper and the GWL in accordance with embodiments herein.
Figures 8A-8C illustrate views of the sensor and catheter positioned in tandem, wherein the distal loop is secured to the GWL with floss and rubber bumpers in accordance with embodiments herein.
Figure 9 illustrates a sheathed delivery system configured to deliver the sensor in accordance with embodiments herein.
Figures 10A-10B illustrate the sensor in a loader tube attached to a threaded delivery cable in accordance with embodiments herein.
Figure 10C illustrates the loader tube, with the sensor therewithin, attached to the delivery sheath in accordance with embodiments herein.
Figure 10D illustrates the sensor exiting the distal end of the delivery sheath in simulated use in accordance with embodiments herein.
Figures 11A-11D illustrate a hybrid suture floss cut release that does not utilize a GWL in accordance with embodiments herein.
Figure 11E illustrates a view of the push rod, window tube, and bumper in accordance with embodiments herein.
Figure 11F illustrates another view of the window tube and bumper, as well as the floss in accordance with embodiments herein.
Figures 11G and 11H illustrate an alternative embodiment that can sever the floss attachment to the sensor without requiring the full length of floss to be pulled to release the sensor in accordance with embodiments herein.
Figure 11I illustrates a sensor delivery system having a cross-sectional view of a lumen configured to hold the GWL, that is held in parallel with the push rod and cutter, and positioned in tandem with the sensor in accordance with embodiments herein.
Figure 11J shows a cross-section of the pressure sheath, the lumen and the cutter tube in accordance with embodiments herein.
Figure 11K is a view of a portion of a tri-lumen catheter and Figure 11L illustrates a cross-sectional view of a sensor delivery system that utilizes the tri-lumen catheter to deliver the sensor within the body in accordance with embodiments herein.
Figure 11M illustrates a top view of a portion of the sensor delivery system in advance of releasing the distal loop in accordance with embodiments herein.
Figure 11N is a view showing the sensor, push rod, cutter tube, window tube, and bumper of the sensor delivery system of Figure 11K in accordance with embodiments herein.
Figure 11O is a view showing the floss entering the opening of the window tube in accordance with embodiments herein.
Figure 11P is a cut-away end view showing the tri-lumen catheter in accordance with embodiments herein.
Figure 11Q illustrates another view of a sensor delivery system that utilizes the tri-lumen catheter to deliver the sensor within the body in accordance with embodiments herein.
Figure 12A is a cross-sectional view of a push rod with the floss extending therethrough and removably coupling the sensor in accordance with embodiments herein.
Figure 12B illustrates the sensor having a ring securely extending through a hole in the sensor in accordance with embodiments herein.
Figure 12C illustrates the sensor wherein the floss extends through the hole in the sensor in accordance with embodiments herein.
Figures 12D-12F illustrate views of a floss loop configured to orient the floss in a horizontal direction in accordance with embodiments herein.
Figures 12G-12I illustrate views of another floss loop configured to orient the floss in a vertical direction in accordance with embodiments herein.
Figure12J illustrates another view wherein an attachment feature can be mounted within the existing holes (e.g., attachment points as shown in Figure 5A) in the sensor that secure the anchor loops (not shown) in accordance with embodiments herein.
Figures 13A-13E illustrate additional embodiments wherein a threaded end of a threaded push rod is protected by a sleeve or shroud after the sensor is delivered in accordance with embodiments herein.
Figures 14A-14D illustrate embodiments wherein a snare contained within a push rod interconnects with a protruding feature on the sensor in accordance with embodiments herein.
Figures 14E and 14F show a snare within a pushrod that includes multiple detent paddles in accordance with embodiments herein.
Figure 14G shows the detent paddles engaged with the protrusion in accordance with embodiments herein.
Figure 14H shows the detent paddles disengaged from the protrusion of the sensor in accordance with embodiments herein.
Figures 15A-15G illustrate embodiments wherein a locking feature at a distal end of push rod can be removably coupled to a pocket attached to and/or integral with the proximal end of the sensor in accordance with embodiments herein.
Figures 16A and 16B illustrate a push rod with a sleeve or sheath around wires that are attached to, integral with, and/or terminate with clamp arms in accordance with embodiments herein.
Figure 16C illustrates a feature attached to one end of the sensor that has recesses formed on opposite sides configured to receive jaws or paddles in accordance with embodiments herein.
Figures 16D-16F illustrate flat paddles, round balls, and/or jaws that are configured to interface with the recesses when the sleeve is pushed forward to lock the features together in accordance with embodiments herein.
Figures 16G-16H illustrate a feature attached to and/or integral with one end of the sensor that has windows or openings on either side of the feature for accepting slides in accordance with embodiments herein.
Figures 161-16L illustrate further embodiments for securing the sensor to the push rod until deployment in accordance with embodiments herein.

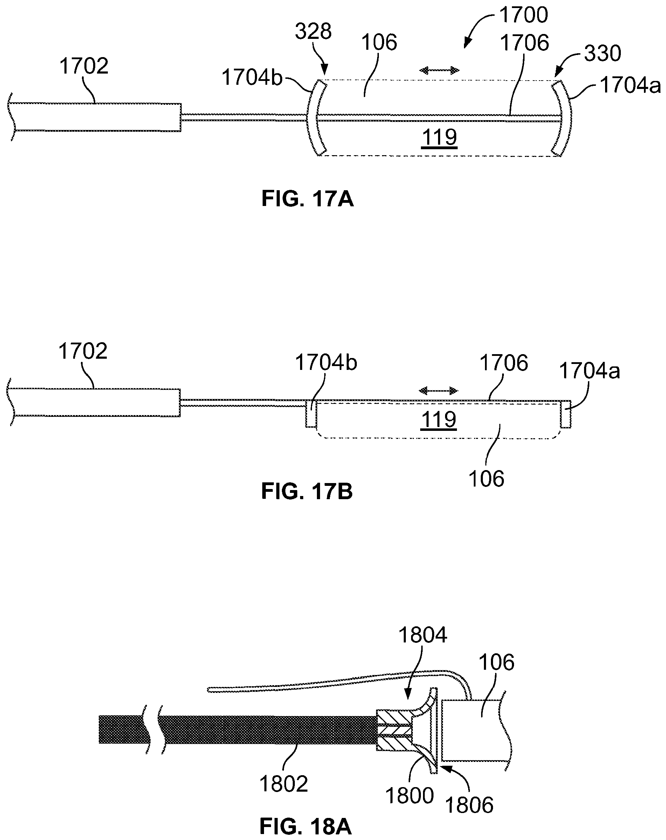

Figures 17A-17B illustrate a pinch clamp mechanism configured to removably couple the sensor to the push rod in accordance with embodiments herein.
Figures 18A-18D illustrate additional embodiments for bumpers and catheters for use supporting sensor during delivery or when retrieving a sensor in accordance with embodiments herein.

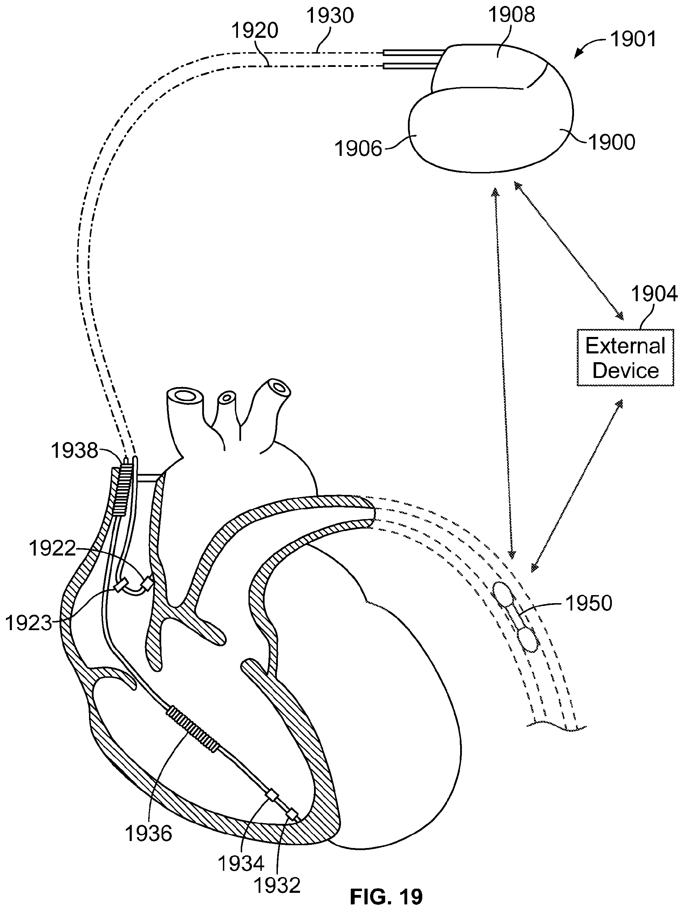

Figure 19 illustrates a system that includes an implantable medical device (IMD), an implantable sensor, and an external device implemented in accordance with embodiments herein.

### DETAILED DESCRIPTION

It will be readily understood that the components of the embodiments as generally described and illustrated in the Figures herein, may be arranged and designed in a wide variety of different configurations in addition to the described example embodiments. Thus, the following more detailed description of the example embodiments, as represented in the Figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

Embodiments may be implemented in connection with concepts describe in the following patents: US Patent 10,653,859, titled "Delivery Catheter Systems and Methods" having an issue date of 05/19/2020, U.S. Patent 10,894,144, titled "Apparatus and method for sensor deployment and fixation" having an issue date of 01/19/2021, and US Patent 11179048, titled "System for deploying an implant assembly in a vessel" having an issue date of 11/23/2021.

Embodiments herein may be implemented in connection with the concepts described in the following patents and applications: US Patent 10433764B2, titled "Implantable sensor enclosure with thin sidewalls" having an issue date of 10/09/2019 ; US Patent 9867552, titled "Implantable sensor enclosure with thin sidewalls" having an issue date of 09/16/2018; US Patent 9538958B2, titled "Permittivity shielding" having an issue date of 01/10/2017; US Patent 8894582B, titled "Cardiac pressure monitoring device" having an issue date of 11/25/2014; US Patent 7677107B2, titled "Wireless pressure sensor and method for fabricating wireless pressure sensor for integration with an implantable device" having an issue date of 03/16/2010; US Patent Application 20200260991A1, titled "Pressure sensing implant" having a publication date of 08/20/2020; US Patent Application 20190200928A1, titled "Pressure sensing implant" having an issue date of 07/04/2019; US Patent Application 20180116552A1, titled "IMPLANTABLE SENSOR ENCLOSURE WITH THIN SIDEWALLS" having a publication date of 05/03/2018; US Patent Application 20160324443A1, titled "PRESSURE SENSING IMPLANT" having a publication date of 11/10/2016; US Patent Application 20160029956A1, titled "PRESSURE SENSING IMPLANT" having a publication date of 02/04/2016; US Patent Application 20140155710A1, titled "IMPLANTABLE SENSOR ENCLOSURE WITH THIN SIDEWALLS" having an issue date of 06/05/2014; US Patent Application 20090024042A1, titled "METHOD AND SYSTEM FOR MONITORING VENTRICULAR FUNCTION OF A HEART" having a publication date of 01/22/2009; US Patent 20090007679A1, titled "WIRELESS PRESSURE SENSOR AND METHOD FOR FABRICATING WIRELESS PRESSURE SENSOR FOR INTEGRATION WITH AN IMPLANTABLE DEVICE" having an issue date of 1/08/2009; US Patent Application 20090011117A1 titled "METHODS FOR TEXTURING A SURFACE OF AN ENDOVASCULAR IMPLANT" having a publication date of 01/08/2009; US Patent 8,382,677 titled "An anchored implantable pressure monitor" having an issue date of 02/26/2013; US Patent 8355777 titled "Apparatus and method for sensor deployment and fixation" having an issue date of 01/15/2013; US Patent 8353841 titled "Apparatus and method for sensor deployment and fixation" having an issue date of 01/15/2013; US Patent 8118749 titled "Apparatus and method for sensor deployment and fixation" having an issue date of 02/21/2012; US Patent 8021307 titled "Apparatus and method for sensor deployment and fixation" having an issue date of 09/20/2011; US Patent 7966886 titled "Method and apparatus for measuring pressure inside a fluid system" having an issue date of 06/28/2011; US Patent 7936174 titled "Coupling loop" having an issue date of 05/03/2011; US Patent 7595647 titled "Cable assembly for a coupling loop" having an issue date of 09/29/2009; US Patent 7432723 titled "Coupling loop" having an issue date of 10/07/08; US Patent 8111150 titled "Physiological data acquisition and management system for use with an implanted wireless sensor" having an issue date of 02/07/2012; US Patent 8159348 titled "Communication system with antenna box amplifier" having an issue date of 04/17/2012; US Patent 8237451 titled "Communicating with an implanted wireless sensor" having an issue date of 08/07/2012; US Patent 7839153 titled "Communicating with an implanted wireless sensor" having an issue date of 11/23/2010; US Patent 7679355 titled "Communicating with an implanted wireless sensor" having an issue date of 03/16/2010; US Patent 7550978 titled "Communicating with an implanted wireless sensor" having an issue date of 06/23/2009; US Patent 7498799B2 titled "Communicating with an implanted wireless sensor" having an issue date of 03/03/2009; US Patent 7466120B2 titled "Communicating with an implanted wireless sensor" having an issue date of 12/16/2008; US Patent 7439723B2 titled "Communicating with an implanted wireless sensor" having an issue date of 10/21/2008; US Patent 7245117B1 titled "Communicating with implanted wireless sensor" having an issue date of 07/17/2007; US Patent 8669770B2 titled "Selectively actuating wireless, passive implantable sensor" having an issue date of 03/11/2014; US Patent 8159348B2 titled "Communication system with antenna box amplifier" having an issue date of 04/17/2012; US Patent 7932732B2 titled "Preventing a false lock in a phase lock loop" having an issue date of 04/26/2011; US Patent 7710103B2 titled "Preventing false locks in a system that communicates with an implanted wireless sensor" having an issue date of 05/04/2010; US Patent 7492144 titled "Preventing false locks in a system that communicates with an implanted wireless sensor" having an issue date of 02/17/2009; US Patent 10143388 titled "Method of manufacturing implantable wireless sensor for pressure measurement" having an issue date of 12/04/2018; US Patent 9792469 titled "Wireless physical property sensor with digital communications" having an issue date of 10/17/2017; US Patent 9653926 titled "Physical property sensor with active electronic circuit and wireless power and data transmission" having an issue date of 05/16/2017; US Patent 9041416 titled "Physical property sensor with active electronic circuit and wireless power and data transmission" having an issue date of 05/26/2015; US Patent 8,264,240 titled "Physical property sensor with active electronic circuit and wireless power and data transmission" having an issue date of 09/11/2012; US Patent 7621036 titled "Method of manufacturing implantable wireless sensor for in vivo pressure measurement" having an issue date of 11/24/2009; US Patent 7574792 titled "Method of manufacturing an implantable wireless sensor" having an issue date of 08/18/2009; US Patent 8360984 titled "Hypertension system and method" having an issue date of 01/29/2013; US Patent 8264240B2 titled "Physical property sensor with active electronic circuit and wireless power and data transmission" having an issue date of 09/11/2012; US Patent 8025625 titled "Sensor with electromagnetically coupled hermetic pressure reference" having an issue date of 09/27/2011; US Patent 7909770 titled "Method for using a wireless pressure sensor to monitor pressure inside the human heart" having an issue date of 03/22/2011; US Patent 7908018 titled "Flexible electrode" having an issue date of 03/15/2011; US Patent 7854172 titled "Hermetic chamber with electrical feedthroughs" having an issue date of 12/21/2010; US Patent 7662653 titled "Method of manufacturing a hermetic chamber with electrical feedthroughs" having an issue date of 02/16/2010; US Patent 7647836 titled "Hermetic chamber with electrical feedthroughs" having an issue date of 01/19/2010; US Patent 7829363 titled "Method and apparatus for microjoining dissimilar materials" having an issue date of 11/09/2010; US Patent 7812416 titled "Methods and apparatus having an integrated circuit attached to fused silica" having an issue date of 10/12/2010; US Patent 7748277 titled "Hermetic chamber with electrical feedthroughs" having an issue date of 07/06/2010; US Patent 7699059 titled "Implantable wireless sensor" having an issue date of 04/20/2010; US Patent 7667547 titled "Loosely-coupled oscillator" having an issue date of 02/23/2010; US Patent 7481771 titled "Implantable wireless sensor for pressure measurement within the heart" having an issue date of 01/27/2009; US Patent 6855115 titled "Implantable wireless sensor for pressure measurement within the heart" having an issue date of 02/15/2005; US Patent 7147604 titled "High Q factor sensor" having an issue date of 12/12/2006; US Patent Application 20200146562 titled "Sensor delivery system and method" having a publication date of 05/14/2020; US Patent Application 20150208929 titled "PRESSURE SENSOR, ANCHOR, DELIVERY SYSTEM AND METHOD" having a publication date of 7/30/2015; US Patent 8,382,677 titled "An anchored implantable pressure monitor" having an issue date of 02/26/2013; US Patent 10282571 titled "Self test device and method for wireless sensor reader" having an issue date of 05/07/2019; US Patent 9996712 titled "Self test device and method for wireless sensor reader" having an issue date of 06/12/2018; US Patent 20170061168 titled "SELF TEST DEVICE AND METHOD FOR WIRELESS SENSOR READER" having an issue date of 03/02/2017; US Patent 10003862 titled "Wireless sensor reader" having an issue date of 06/29/2018; US Patent 9894425 titled "Wireless sensor reader" having an issue date of 02/23/2017; US Patent 9721463 titled "Wireless sensor reader" having an issue date of 08/01/17; US Patent 9489831 titled "Wireless sensor reader" having an issue date of 11/08/2016; US Patent 9305456 titled "Wireless sensor reader" having an issue date of 04/05/2016; US Patent 8570186 titled "Wireless sensor reader" having an issue date of 10/29/2013; US Patent 8493187 titled "Wireless sensor reader" having an issue date of 07/23/2013; US Patent 8432265 titled "Wireless sensor reader" having an issue date of 4/30/2013; US Patent 8154389 titled "Wireless sensor reader" having an issue date of 04/10/2012; US Patent 7839153 titled "Communicating with an implanted wireless sensor" having an issue date of 11/23/2010; US Patent 7679355 titled "Communicating with an implanted wireless sensor" having an issue date of 3/16/2010; US Patent Application 20170309164 titled "WIRELESS SENSOR READER" having a publication date of 10/26/2017; US Patent Application 20170055048 titled "WIRELESS SENSOR READER" having a publication date of 02/23/2017; US Patent Application 20160210846 titled "WIRELESS SESOR READER" having a publication date of 7/21/2016; US Patent Application 20140306807 titled WIRELESS SENSOR READER having a publication date of 10/16/2014; US Patent Application 20140028467 titled "WIRELESS SENSOR READER" having a publication date of 01/30/2014; US Patent Application 20130222153 titled "WIRELESS SENSOR READER" having a publication date of 08/29/2013; US Patent Application 20090189741 titled "WIRELESS SENSOR READER" having a publication date of 07/30/2009; US Patent 8,382,677 titled "An anchored implantable pressure monitor" having an issue date of 02/26/2013; US Patent 10003862 titled "Wireless sensor reader" having an issue date of 06/29/2018; US Patent 9894425 titled "Wireless sensor reader" having an issue date of 2/13/2018; US Patent 9721463 titled "Wireless sensor reader" having an issue date of 08/01/2017; US Patent 9489831 titled "Wireless sensor reader" having an issue date of 11/08/2016; US Patent 9305456 titled "Wireless sensor reader" having an issue date of 04/05/2016; US Patent 8570186 titled "Wireless sensor reader" having an issue date of 10/29/2013; US Patent 8493187 titled "Wireless sensor reader" having an issue date of 07/23/2013; US Patent 8432265 titled "Wireless sensor reader" having an issue date of 04/30/2013; US Patent 8154389 titled "Wireless sensor reader" having an issue date of 04/10/2012; US Patent Application 20170309164 titled "WIRELESS SENSOR READER" having a publication date of 10/26/2017; US Patent Application 20170055048 titled "WIRELESS SENSOR READER" having a publication date of 02/23/2017; US Patent Application 20160210846 titled "WIRELESS SENSOR READER" having a publication date of 7/21/2016; US Patent Application 20140306807 titled "WIRELESS SENSOR READER" having an issue date of 10/16/2014; US Patent Application 20140028467 titled "WIRELESS SENSOR READER" having a publication date of 1/30/2014; US Patent Application 20130222153 titled "WIRELESS SENSOR READER" having a publication date of 8/29/2013; US Patent Application 20090189741 titled "WIRELESS SENSOR READER" having a publication date of 7/30/09; US Patent 9867552 titled "Implantable sensor enclosure with thin sidewalls" having an issue date of 01/16/2018; US Patent Application 20180116552 titled "IMPLANTABLE SENSOR ENCLOSURE WITH THIN SIDEWALLS" having a publication date of 5/03/2018; US Patent Application 20160324443 titled "PRESSURE SENSING IMPLANT" having a publication date of 11/10/2016; US Patent Application 20160029956 titled "PRESSURE SENSING IMPLANT" having a publication date of 02/04/2016; US Patent Application 20140155710 titled "IMPLANTABLE SENSOR ENCLOSURE WITH THIN SIDEWALLS" having a publication date of 6/05/2014; US Patent 9538958 titled "Permittivity shielding" having an issue date of 1/10/2017; US Patent 8894582 titled "Cardiac pressure monitoring device" having an issue date of 11/25/2014; US Patent Application 20090024042 titled "METHOD AND SYSTEM FOR MONITORING VENTRICULAR FUNCTION OF A HEART" having a publication date of 1/22/2009; US Patent 7677107 titled "Wireless pressure sensor and method for fabricating wireless pressure sensor for integration with an implantable device" having an issue date of 3/16/2010; US Patent Application 20090007679 titled "WIRELESS PRESSURE SENSOR AND METHOD FOR FABRICATING WIRELESS PRESSURE SENSOR FOR INTEGRATION WITH AN IMPLANTABLE DEVICE" having a publication date of 1/18/2009; US Patent Application 20090011117 titled "METHODS FOR TEXTURING A SURFACE OF AN ENDOVASCULAR IMPLANT" having a publication date of 1/08/2009; US Patent Application 20150208929 titled "PRESSURE SENSOR, ANCHOR, DELIVERY" having a publication date of 7/30/2015; US Patent Application 20090009332 titled "SYSTEM AND METHOD FOR MONITORING INGESTED MEDICATION VIA RF WIRELESS TELEMETRY" having a publication date of 01/08/2009; and US Patent 8,382,677 titled "An anchored implantable pressure monitor" having an issue date of 2/26/2013.

### Terms

The term "skive" shall mean an area of removed material, such as an opening or notch in a catheter that is accessible from outside the catheter. The terms "skiving" and "skived" shall mean removing a portion of the catheter material, such as to expose a lumen within the catheter and/or to create an opening for a component (e.g., a portion of an anchor loop, a thread, floss, etc.) to pass through and/or be secured.

The term "tandem" shall mean one behind another and/or end to end in a locked manner. For example, a sensor and a delivery catheter can be held in tandem by locking the sensor to a "rail", such as a guidewire lumen that extends through the delivery catheter.

The term "in parallel" and "parallel" shall mean one on top of the other and/or positioned side-by-side.

The term "intracorporeal sensor" shall mean any sensor configured to be inserted and fixed within a body. In some cases, the intracorporeal sensor is a pressure sensor.

The terms "push rod" and "push catheter" shall mean any wire, rod, and/or assembly of components such as rod(s), sheaths, and the like that push a sensor within a sheath (e.g., delivery sheath) to a delivery location within a body.

The term "attachment feature" shall mean any feature associated with and/or attached to and/or integral with the sensor that can be used to removably couple the sensor to one or more components of the delivery system.

The term "release mechanism" shall mean any feature associated with and/or attached to and/or integral with the delivery system that can be used to removably couple the one or more components of the delivery system to the sensor.

The terms "catheter" and "delivery catheter" shall mean any catheter or generally smooth cylindrical body that encloses one or more lumen and is configured to be inserted into a body. The catheter can include a single opening or lumen in which one or more features extend, multiple lumens in which each lumen includes zero features, such as a lumen used to measure pressure, or one or more features. The term catheter shall also mean a pressure sheath when a pressure sheath is used to enclose one or more features and is configured to be inserted into a body.

### Tandem Interlace Delivery Catheter Overview

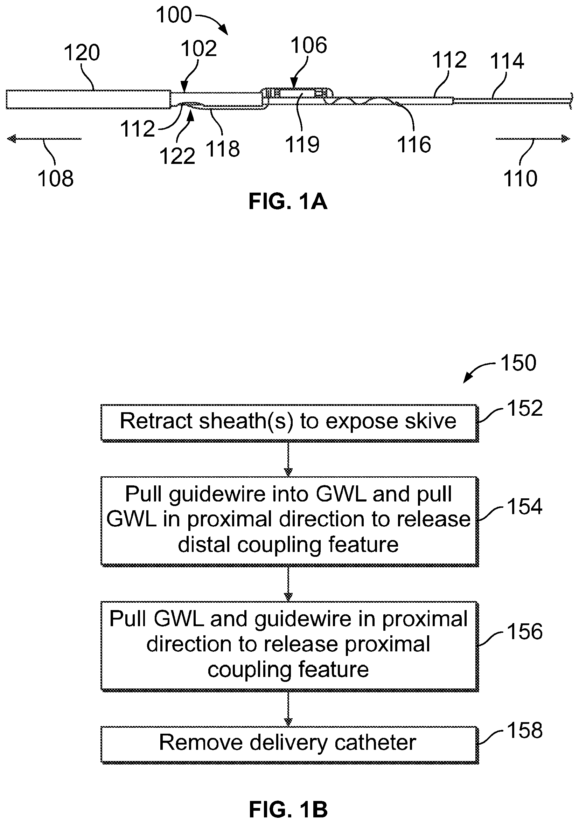

Figure 1A illustrates an intracorporeal sensor delivery system 100 in accordance with embodiments herein. A delivery catheter 102 is positioned in tandem to an intracorporeal sensor 106. For reference, proximal direction 108 and distal direction 110 are indicated. For example, proximal direction 108 or orientation indicates the portion of the system 100 closest to the practitioner.

The catheter 102 can be a single lumen catheter or a multi-lumen catheter as discussed further below. A guidewire lumen (GWL) 112 extends within the catheter 102, such as within one lumen of the multi-lumen catheter or within the single lumen catheter. A guidewire 114 can extend within the GWL 112.

The sensor 106 can be a pressure sensor or can be a sensor 106 used to detect other indications within the body. In some embodiments, the pressure sensor 106 can be positioned within the pulmonary artery and be configured to be able to communicate with an implantable medical device, such as a pacemaker, etc., and/or communicate with an external device.

The sensor 106 can utilize one or more anchor mechanisms, such as loops, to secure the sensor 106 in place within the vessel when the sensor 106 is deployed at a deployment location. One or more of the anchor mechanisms can be used to removably couple the sensor 106 to the sensor delivery system 100. This provides an advantage over previous delivery systems as additional wires or other attachments are no longer needed to removably couple the sensor 106 to the sensor delivery system 100. A distal loop 116 and a proximal loop 118 can be attached to a body 119 of the sensor 106. The distal loop 116 (e.g., distal coupling feature) of the sensor 106 interfaces with an outer surface of the GWL 112. An interlacing weave pattern or other twisting, wrapping, etc., can be used to position the distal loop 116 around the outer surface of the GWL 112 as discussed further below. A portion of the proximal loop 118 (e.g., proximal coupling feature) can be retained between the GWL 112 and the catheter 102. For example, a first side of a proximal loop 118 extends into and/or through an opening or skive 122 in the catheter 102. The proximal loop 118 is positioned between the GWL 112 and an inner portion of the catheter 102 and/or the skive 122. A second side of the proximal loop 118 exits through the skive 122 on an opposite side of the GWL 112. Therefore, the sensor 106 is secured and/or interlocked to the catheter 102 while the GWL 112 is in place (e.g., while the GWL 112 extends through the portion of the catheter 102 coinciding with the skive 122 and the proximal loop 118). The distal and proximal loops 116, 118 (e.g., anchor loops) can be formed of Nitinol or other shape retaining material as is known in the art. Although the sensor 106 is shown as having two anchor loops, in some embodiments, the sensor 106 may include only the distal loop 116 or the proximal loop 118, or may include one or more additional loops.

A pressure sheath 120 can extend over the catheter 102 and portions of the GWL 112. In some embodiments a pressure sheath 120 is not utilized. In other embodiments, an introducer sheath (not shown) or other sheath (e.g., separate lumen) can extend over the catheter 102, sensor 106, GWL 112 and/or pressure sheath 120, and/or be integrated with or positioned alongside one or more of the catheter 102, sensor 106, GWL 112, and pressure sheath 120.

In some embodiments, the distal loop 116 and/or proximal loop 118 can be interlaced, wrapped, twisted together, combinations thereof, etc., around the outer surface of the GWL 112 to removably couple the distal and proximal loops 116, 118 to the GWL 112. Thus, in response to the GWL 112 being decoupled from the coupling feature, the distal and proximal loops 116, 118 will open to engage walls of a vessel. In some embodiments, the distal and/or proximal loops 116, 118 will open laterally with no axial twisting and the opened distal and proximal loops 116, 118 will engage walls of the vessel to provide rotational and axial stability of the sensor 106.

Figure 1B illustrates a method 150 for releasing the sensor 106 within a vessel after the sensor 106 is positioned at the desired location in accordance with embodiments herein. At 152, the pressure sheath 120 and/or other outer sheath(s) are at least partially retracted in the proximal direction 108, as shown in Figure 1A, such that the sheath(s) are proximally located with respect the skive 122. It should be noted that in some embodiments, the retraction of the one or more sheaths can be accomplished after the distal loop 116 (e.g., coupling feature) is released at 154. In some embodiments, if the pressure sheath 120 is not used, no retraction or partial retraction is needed.

At 154, the guidewire 114 is pulled into the GWL 112, and then the GWL 112 is pulled in the proximal direction 108. As the practitioner pulls the GWL 112 (and guidewire 114 if still within GWL 112) in the proximal direction 108, the GWL 112 slides through the interlaced distal loop 116. The distal loop 116 is released from the sensor delivery system 100 and pops open to interface with walls of the patient's vessel. An advantage of the sensor delivery system 100 compared to previous delivery systems is that there is no catheter material distal with respect to the sensor 106 when the distal loop 116 is released.

At 156, the GWL 112 (and guidewire 114) are pulled in the proximal direction 108 to release the proximal loop 118 (e.g., coupling feature) from the catheter 102. When the proximal loop 118 is released, the sensor 106 is separated from the delivery system 100. In some embodiments, the proximal loop 118 may release from the delivery system 100 when less than the entire skive 122 is exposed.

At 158, the delivery catheter 102 is pulled in the proximal direction 108. When the GWL 112 is clear of the skive 122, the proximal loop 118 is released and will pop fully open to interface with the patient's vessel.

Further, the pressure sheath 120 and/or other sheaths can be retracted during the release of the sensor 106 or before the GWL 112 is pulled in the proximal direction 108 to release the distal loop 116. However, the pressure sheath 120 can remain near the deployed sensor 106 to allow pressure readings to be taken. In other embodiments, the catheter 102 can remain in place if the catheter 102 has its own pressure lumen (e.g., multi-lumen catheter). In still further embodiments, adequate clearance of e.g., approximately 0.04 square inch may be provided within the catheter 102 to facilitate pressure readings. Also, the steps of the method may occur in a different order. For example, the GWL 112 and guidewire 114 may be pulled to free the distal and proximal loops 116, 118 before the sheath(s) are retracted.

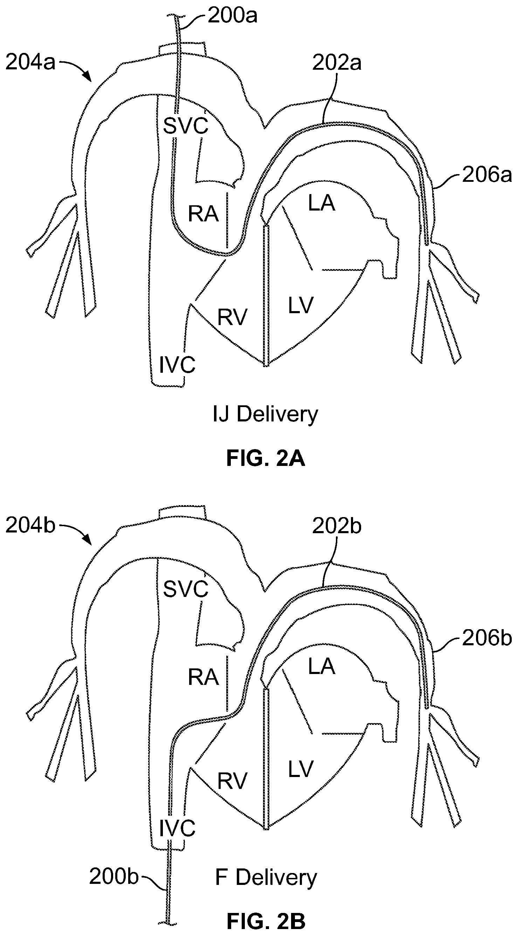

Figures 2A and 2B illustrate delivery paths 200a, 200b that can be used to position a delivery sheath 202a, 202b, and thus also the sensor delivery system 100, in place within a patient 204a, 204b in accordance with embodiments herein. In this example, the sensor 106 is a pressure sensor 106 and is located within pulmonary artery 206a, 206b. Figure 2A shows the insertion of the delivery sheath 202a from internal jugular access (IJ Delivery), through the Superior Vena Cave (SVC). Figure 2B shows the insertion of the delivery sheath 202b through femoral access (F Delivery) through the Inferior Vena Cava (IVC).

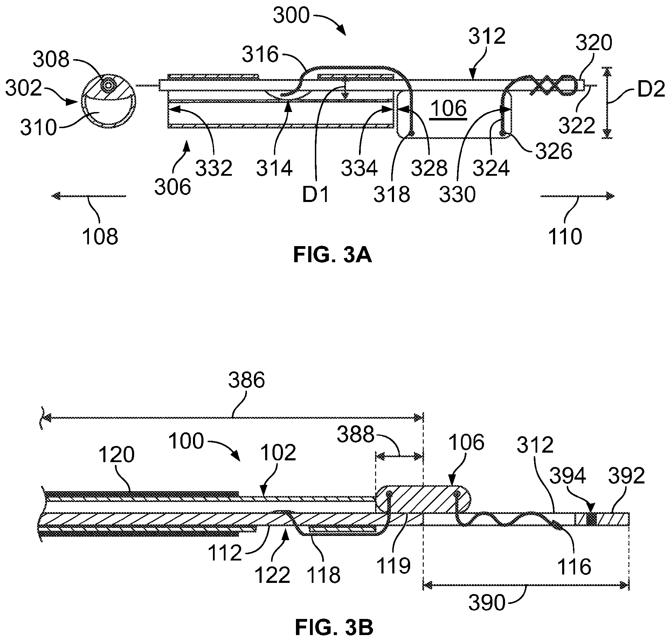

Figure 3A illustrates a cross-sectional view of sensor delivery system 300 and a cross sectional view 302 of a multi-lumen catheter 306 in accordance with embodiments herein. A sensor 106 is in tandem with the multi-lumen catheter 306, wherein the sensor 106 is distal with respect to the catheter 306. In other words, the sensor 106 has a proximal end 328 and a distal end 330. Similarly, the catheter 306 has a proximal end 332 and a distal end 334.

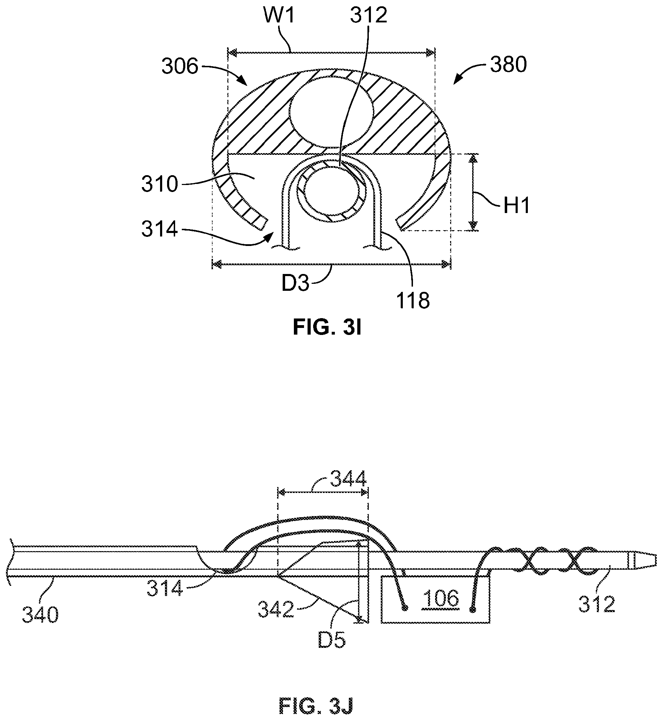

The multi-lumen catheter 306 includes first and second lumens 308, 310 extending therethrough. The first lumen 308 receives GWL 312, through which guidewire 322 is removably inserted. In the embodiment shown, the second lumen 310 can be empty or can receive other components not shown, such as to measure pressure (e.g., pulmonary arterial pressure (PAP)), etc. Although the first lumen 308 is shown as circular and the second lumen 310 is shown as having an approximate "D" shape, the first and second lumens 308, 310 can be other shapes to accommodate the uses of the lumen. Further, the first and second lumens 308, 310 can be different sizes than shown. For example, the first lumen 308 may encompass a larger cross-sectional area to accommodate a larger GWL 312 and/or the first and second lumens 308, 310 can be approximately the same size and/or same shape. The area of the multi-lumen catheter 306 around the first and second lumens 308, 310 can include catheter material, additional lumens, delivery or other passages, open space, etc. In other embodiments, the GWL 312 can extend through the second lumen 310, as discussed further below in Figure 3I.

The sensor 106 is substantially in parallel with the GWL 312, which extends beyond the distal end 330 of the sensor 106. The sensor 106 remains distal with respect to the multi-lumen catheter 306. When the sensor delivery system 300 is inserted into an introducer sheath (not shown), the GWL 312 protrudes first, and the sensor 106 is positioned in the introducer sheath before the distal end 334 of the main catheter body. The multi-lumen catheter 306 holds the GWL 312 off center, and thus in some embodiments the GWL 312 and the multi-lumen catheter 306 are positioned to minimize vertical space (e.g., diameter D2), such as from an upper, outer surface of the multi-lumen catheter 306 and/or clearance needed for distal loop 324 to an opposite side of the sensor 106 or an opposite side of the multi-lumen catheter 306, whichever is greater. In some embodiments, the sensor 106 can, at least partially, overlap vertically with a pressure sheath (discussed further below) and inner shaft of the catheter 306. This ensures that the multi-lumen catheter 306 and the sensor 106 can be loaded together and moved through the introducer sheath without interference. Also, by arranging the multi-lumen catheter 306 and the sensor 106 in tandem, a size advantage can be realized as the introducer sheath can have a smaller diameter compared to systems that utilize arrangements that stack the catheter and the sensor on top of each other (e.g., catheter and sensor are held in parallel).

The GWL 312 may have a marker band (discussed further below in Figure 3B) on a distal tip 320 for visibility. The distal tip 320 can be covered with a flexible tip material to prevent vessel puncture. In some embodiments, the GWL 312 may be constructed having an inner layer of solid high-density polyethylene (HDPE), elastomer(s) or plastic such as PEBAX, or nylon outer layer, and can have a lubricant additive such as Propell, Mobilize, etc. The GWL 312 may be made of a multilayer extrusion with a tie layer between a lubricious material and more suitable bonding polymer. An inside layer could be polytetrafluoroethylene (PTFE)/braid/polyimide(PI), PTFE/braid/PI/PEBAX, PI/Braid/PI, HDPE, PE pebax with lubricant additive, etc., while the exterior layer can be nylon, pebax, pebax with lubricant additives, etc.

Figure 3B illustrates another embodiment wherein the GWL 312 has a variable stiffness in accordance with embodiments herein. The variable stiffness GWL 312 can be formed wherein different lengths of the GWL 312 have two or more different stiffnesses. For example, a proximal portion 388 from a proximal manifold to extending 1 - 30 mm distal to the delivery system, or a proximal portion 386 extending a greater portion of the GWL 312, can be reinforced or stiffer than a remaining distal portion 390. This reinforcement can be i) reinforced over the GWL 312, such as a braided polyimide tube bonded exterior to the full or desired length of the polymer tube or ii) the reinforcement can be a PTFE/braid/PI/PEBAX proximal tube welded to (e.g., butt joint, diagonal joint, overlap joint, etc.) a softer polymer distal tube (e.g., the distal portion 390).

In some embodiments, the variable stiffness GWL 312 can utilize a stiffer polymer for the proximal portion 386, 388 of the GWL 312 like the braided PI shafts, PEEK, vestamid, grilamid, 72 durometer (D) or 74D Pebax as a sleeve over a full length softer polymer shaft such as a 70D, 63D, 55D, 40D, Pebax distal or trilayer inner HDPE with soft outer layer. Alternatively, the stiffer polymer proximal portion 386, 388 of the GWL 312, such as a multilayer braided polyimide, vestamid, grilamid, 72D or 74D Pebax, etc., can be coaxially bonded to a softer polymer shaft such as a 70D, 63D, 55D, 40D, Pebax distal or trilayer inner HDPE with soft outer layer, etc., at the distal portion 390 of the GWL 312 which would include an optional bond of a third softer tip portion 392 for the distal portion 390 (e.g., 40 D, 55 D, Pebax, etc.). The markerband 394 can be swaged or bonded on the distal end of the distal soft GWL portion 390 either just proximal to (e.g., 1 mm) or under the proximal section of the soft tip portion 392. Suitable markerband materials are Platinum Iridium, Gold, or Silver, etc. It should be understood that although suitable materials as well as the stiffness or hardness thereof, are suggested herein, other materials not specifically named are also contemplated, and thus the embodiments are not limited to the listed materials and associated stiffnesses.

Returning to Figure 3A, a proximal loop 316 removably couples the sensor 106 to the catheter 306. A portion of the catheter material of the multi-lumen catheter 306 has been removed to form a skive 314. In other embodiments, one or more skive 314 can be formed in a mono-lumen or single lumen catheter (not shown). Although the skive 314 is shown as a partial-moon, half-circle, or arc shape, other geometric shapes can be used. Depth D1 of the skive 314 with respect to an outer surface of the multi-lumen catheter 306 can be determined based on an outer diameter of the GWL 312 as well as an outer diameter of material forming proximal loop 316. In some embodiments, the skive 314 may remove material without extending into another lumen (e.g., the second lumen 310), The proximal loop 316 is attached to two attachment points that can be located within a region close to the proximal end 328 of the sensor 106 (one attachment point 318 is shown). The proximal loop 316 extends in the proximal direction 108 from the attachment points 318, outside the catheter 306, and through the opening created by the skive 314 between the GWL 312 and the catheter material.

The location of the skive 314 can be based on the length of the proximal loop 316, a desired tightness/looseness of the proximal loop 316 when extending between the GWL 312 and the catheter 306, and/or whether the proximal loop 316 is interlaced around an outer surface of the GWL 312 as discussed further below.

Distal loop 324 can have two attachment points that can be located within a region close to the distal end 330 of the sensor 106 (one attachment point 326 is shown). The distal loop 324 is removably coupled to the GWL 312, such as by being "woven" or interlaced around an outer surface of the GWL 312. In some embodiments, the distal loop 324 is twisted or pulled back and forth along the GWL 312 with deliberate alternating twist directions to keep each side of the distal loop 324 un-tangled with the other. The number of twists can be variable, depending on how tight the distal loop 324 should be against the outer surface of the GWL 312, the length of the distal loop 324, etc.

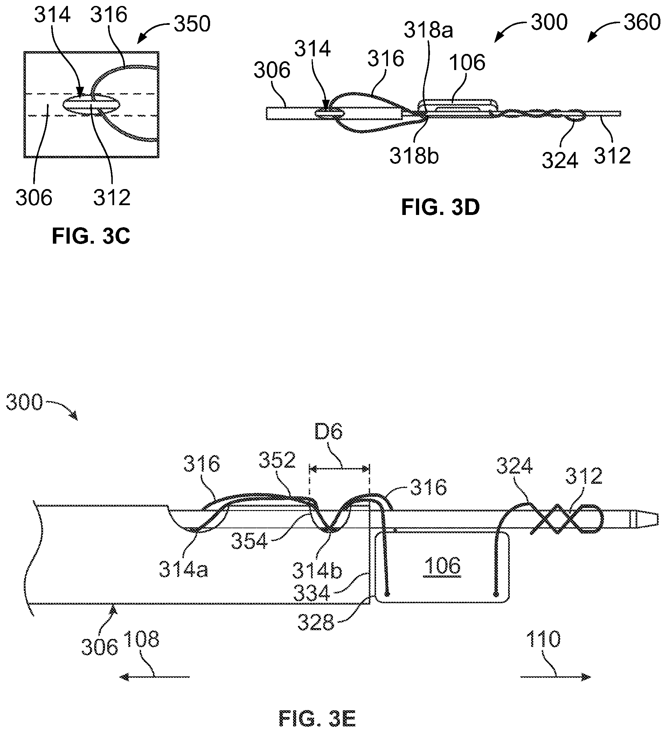

Figure 3C shows a view 350 of the skive 314 in the multi-lumen catheter 306 with the proximal loop 316 captured between the GWL 312 and the skive 314 of the catheter 306 in accordance with embodiments herein. Figure 3D shows a view 360 of the sensor delivery system 300 in accordance with embodiments herein. The proximal loop 316 is captured between the GWL 312 and the skive 314 in the multi-lumen catheter 306. The proximal loop 316 is attached to the sensor 106 at the attachment points 318a, 318b. The distal loop 324 is interlaced around the outer surface of the GWL 312.

In comparison with previous systems, the interlacing of the distal loop 324 around the GWL 312 eliminates the need for sewing or tethering the distal loop 324 and/or proximal loop 316 to multiple skives of the delivery catheter. Accordingly, the assembly process is improved, and less friction is encountered when releasing the sensor 106. Further, the procedure is simplified compared to previous systems because no catheter material, or other delivery system material, is distal to the sensor 106 once the distal loop 324 is released from the GWL 312.

In addition, previous catheter delivery systems having multiple skives required a lubricious coating to be added to portions of the catheter to reduce the friction. By reducing the number of skives, such as by using only one skive 314, to secure the sensor 106 to the catheter 306, the outer surface of the catheter 306 is "smoother". The release is better controlled by one component being pulled through a smoother sheath, rather than through the multiple skives of the catheter. For example, the GWL 312 can be constructed with low friction polymers (e.g., HDPE, PTFE). Therefore, in some cases the additional lubricious coating can be eliminated. In other cases, the materials used for the catheter 306 can be compounded to include lubricious material such as Pebax with Mobilize or Propel, etc.

Figure 3E illustrates a cross-sectional view of the sensor delivery system 300 wherein the multi-lumen catheter 306 includes skives 314a and 314b in accordance with embodiments herein. The proximal loop 316 removably couples the sensor 106 to the catheter 306, and the distal loop 324 removable couples the sensor 106 to the GWL 312.

The proximal loop 316 extends in the proximal direction 108, outside the catheter 306, and through the opening created by the skive 314b between the GWL 312 and the catheter material. For example, both portions of the proximal loop 316 can extend between the GWL 312 and the catheter material, such as to enter and exit the skive 314b along the same side as each other. Additionally or alternatively, the proximal loop 316 can be twisted to retain the proximal loop 316, such that the side portions of the proximal loop 316 enter and exit the skive 314b along opposite side of the GWL 312 as each other.

The proximal loop 316 exits a proximal end 354 of the skive 314b and can be crossed or twisted outside of the catheter 306. In some embodiments, the proximal loop 316 is crossed to form an X 352. The proximal loop 316 then further extends into the skive 314a and is captured between the GWL 312 and the skive 314a.

In some embodiments, a distance D6 from a proximal end 354 of the skive 314b and the distal end 334 of the catheter 306 (or the proximal end 328 of the sensor 106) is less than 10 mm. In some embodiments, the distance D6 is approximately 2 mm. An advantage of the additional skive 314b is that the distal skive 314b keeps the proximal loop 316 from moving up and over the catheter shaft, and the proximal skive 314a locks the remaining length of the proximal loop 316, keeping the sensor 106 from sliding axially.

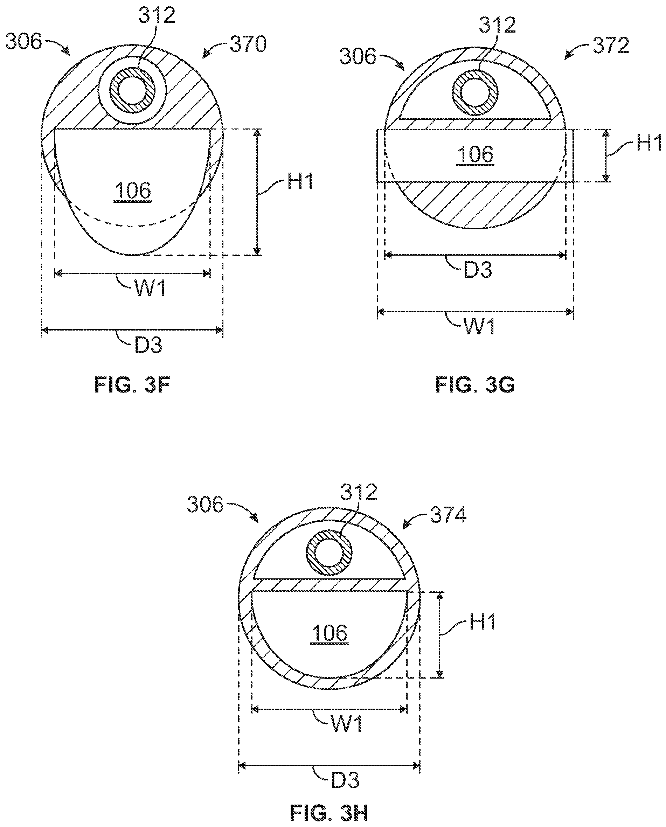

An advantage of the tandem design is the ability to tether/removably couple the sensor 106 to the GWL 312 that has a smaller diameter than the entire catheter 306, allowing the sensor 106 to lie more concentric with the delivery system and save vertical space, thus fitting through smaller introducer sheaths. Figures 3F, 3G, and 3G show end cross-sectional views 370, 372, 374 of the sensor 106 and the multi-lumen catheter 306 positioned in tandem and viewed in the proximal direction 108 in accordance with embodiments herein. The multi-lumen catheter 306 keeps the GWL 312 off center. However, embodiments herein are not limited to the use of a multi-lumen catheter 306 as shown. As shown in Figure 3F, the sensor 106 has width W1 that is within outer diameter D3 of the multi-lumen catheter 306, while height H1 of the sensor 106 extends beyond the outer diameter D3. As shown in Figure 3G, the width W1 of the sensor 106 extends beyond the outer diameter D3 of the multi-lumen catheter 306, while the height H1 of the sensor 106 is within the outer diameter D3. In Figure 3H, the outer diameter D3 of the multi-lumen catheter 306 is sized to accommodate the sensor 106 that has the width W1 and height H1, thus creating an overall concentric type design. In some embodiments the size of the outer diameter D3 can be close to but slightly larger than the size of the sensor 106. In other embodiments, an outer diameter of a distal portion of the catheter shaft can be sized to create the overall concentric type design.

In some embodiments, the tandem design can fit within an 11F sheath (e.g., introducer), which is smaller than the currently used 12F sheath. This provides an advantage wherein the sensor deliver system 100, 300 can be used in smaller vessels and thus a greater variety of patients. In addition, the smaller size can allow for reduced push forces through the 12F or larger sheath, and/or the size of the guidewire 322 could be increased to possibly eliminate a guide wire exchange step in the implantation procedure. In general, a benefit is realized as smaller sized components reduce the risk of patient harm.

The GWL 312 is in parallel with the sensor 106, and the sensor 106 can overlap portions of the multi-lumen catheter 306 vertically and/or horizontally. Also, the sensor 106 may be different shapes, such as substantially rectangular as shown in Figure 3A, have a rounded shape on at least one side as shown in Figures 3F and 3H, or other geometries not shown.

Figure 3I illustrates an end cross-sectional view 380 of the multi-lumen catheter 306 having the GWL 312 extending through the larger second lumen 310 in accordance with embodiments herein. The proximal loop 118 is shown extending into the skive 314, and the GWL 312 securely retains the proximal loop 118.

A funnel or bumper (not shown) can be attached to, for example, either an inner shaft of the catheter 306 or a pressure jacket (not shown) to help center the sensor 106 during one or more of i) loading the sensor 106 and catheter 306 in another sheath, ii) during deployment, and iii) retrieval of the sensor 106 into the catheter 306 if the procedure is aborted. The funnel may prevent sensor damage, allowing the sensor 106 to be re-used. In some embodiments, the funnel can be located to envelop the proximal end 328 of the sensor 106 to keep the sensor 106 taut with the GWL 312 and closer to a concentric position.

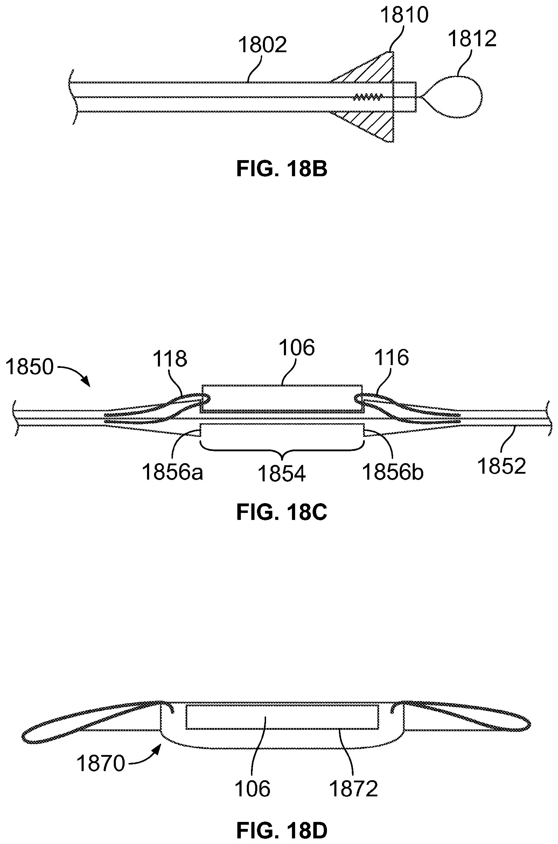

For example, the multi-lumen catheter 306 or a single lumen catheter can include a tapered portion adjacent to the sensor 106, such as to flare outwardly along all or portion(s) of the outer diameter of the catheter. Figure 3J shows a cross-sectional view of a mono-lumen catheter 340 that has an asymmetric bumper 342 with an outer diameter D5 that is larger than the smaller diameter catheter shaft in accordance with embodiments herein. The asymmetric bumper 342 can extend further from the outer surface of the catheter 340 in the area adjacent to the sensor 106. The asymmetric bumper 342 therefore has a nonsymmetric tip portion 344 that creates a closer concentricity with the sensor 106. Other bumpers, such as a symmetrically-shaped bumper, can be used and are discussed further below in Figures 18.

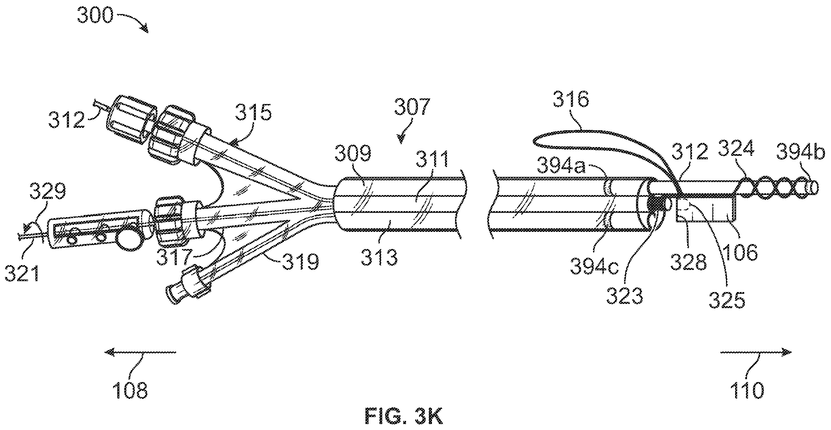

Figure 3K illustrates a sensor delivery system 300 that utilizes a tri-lumen catheter 307 to deliver the sensor 106 within the body in accordance with embodiments herein. The catheter 307 includes three lumens, a first lumen referred to herein as a GWL lumen 309 that accepts the GWL 312, a second lumen referred to herein as a torque cable lumen 311 that accepts a torque cable 321, and a third lumen referred to herein as a pressure lumen 313 that facilitates measuring pressure. Located at a proximal terminal end of the catheter 307 is a guidewire lumen hub 315, a torque cable hub 317, and a pressure lumen hub 319.

At the distal end of the catheter 307, in some cases, the GWL lumen 309 can have marker band 394a. The GWL 312 extends from the GWL lumen 309 and can optionally have a marker band 394b. Optionally or alternatively the pressure lumen 313 can have marker band 394c.

The distal loop 324 of the sensor 106 can be twisted, interlaced or otherwise wrapped around the GWL 312 as discussed herein. The proximal loop 316 of the sensor 106 may be unattached.

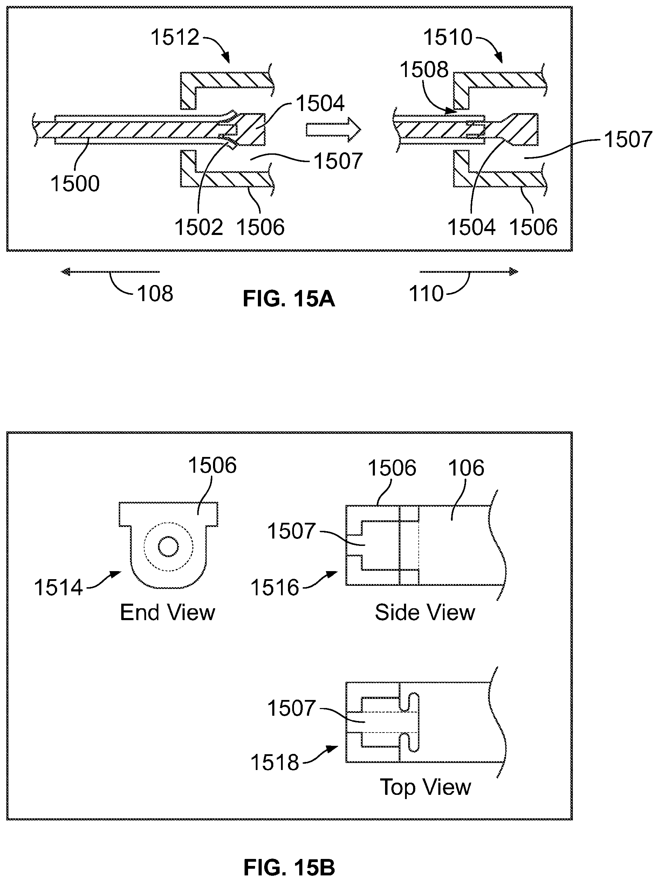

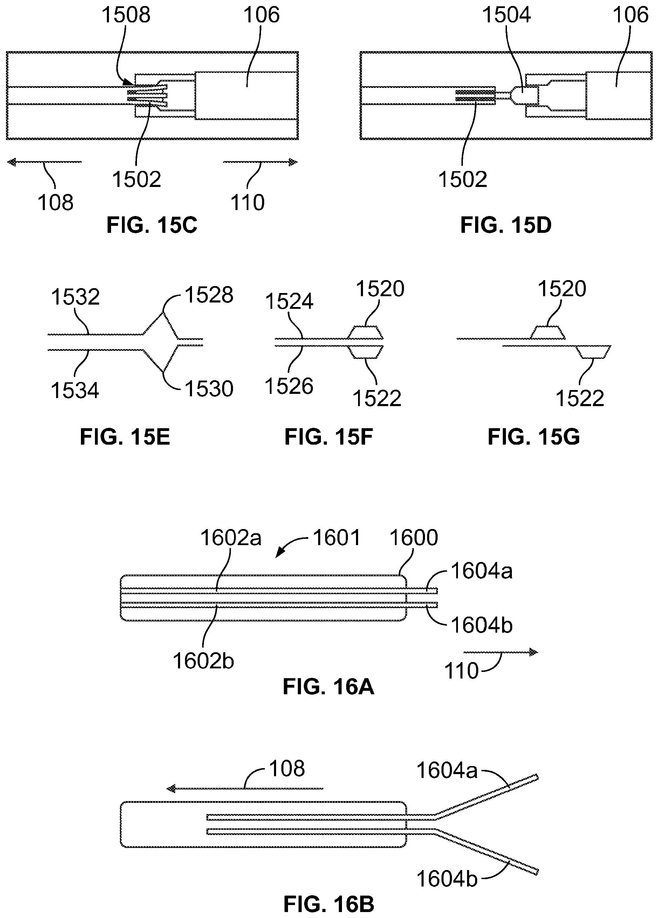

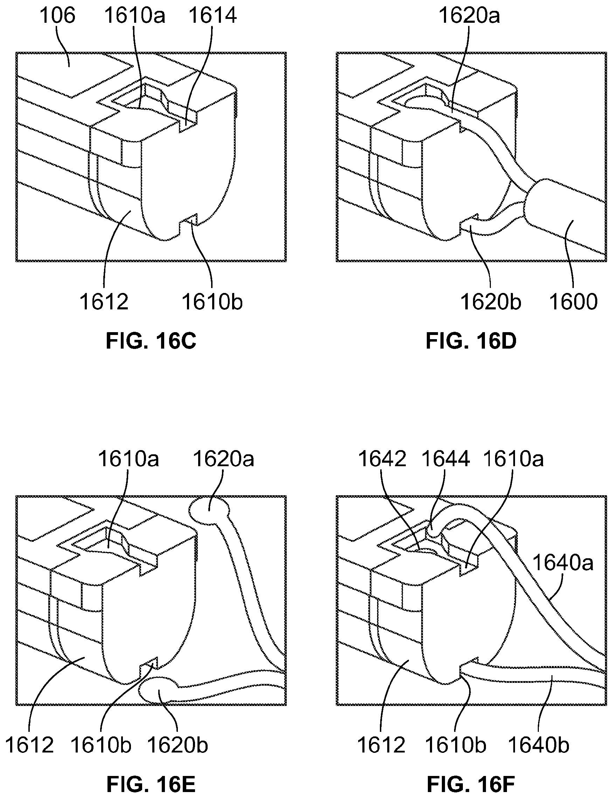

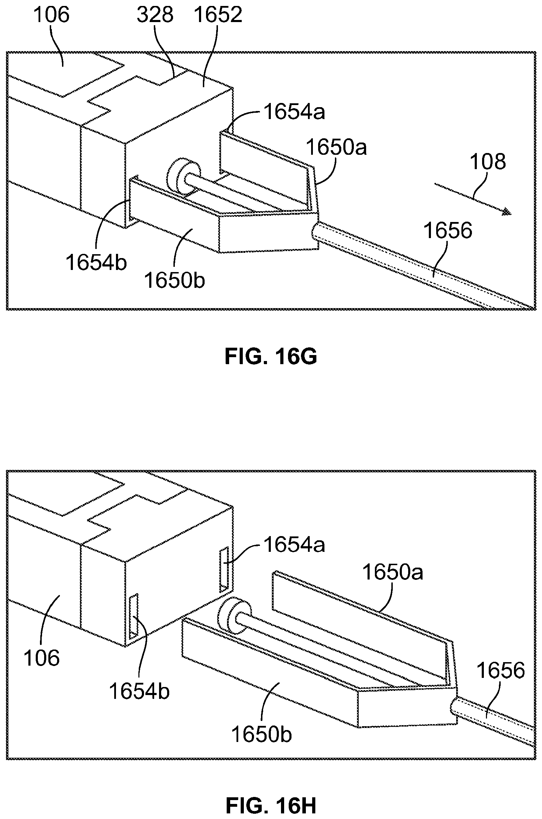

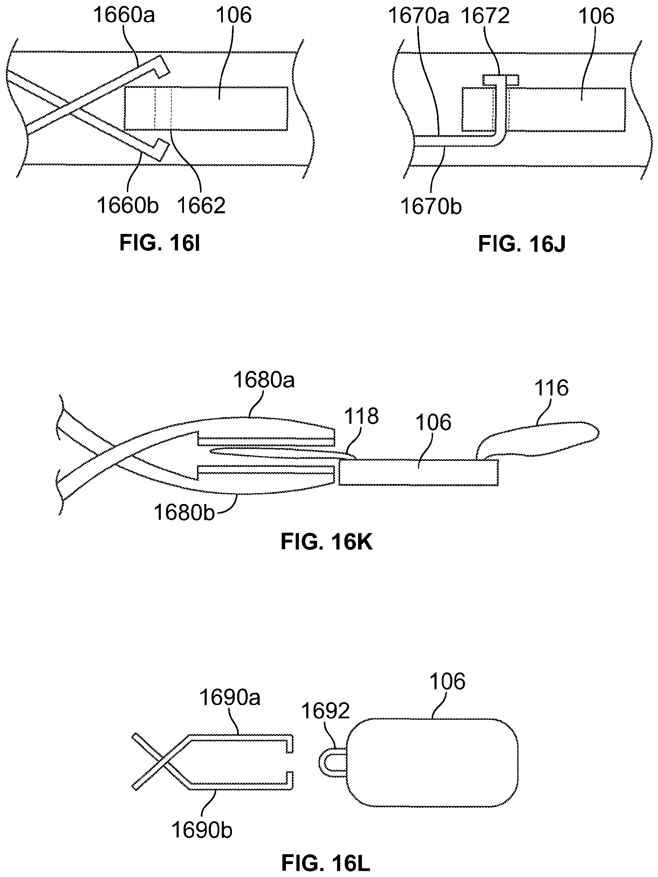

The torque cable 321 includes a threaded fastener 323 that is accepted by a hole 325 in the proximal end 328 of the sensor 106. The threaded fastener 323 is shown in this view as aligned with and outside the hole 325. In some embodiments the threaded fastener 323 can be accepted by a nut at the proximal end 328 of the sensor 106 as discussed further below with respect to Figures 15 and 16.

By way of example, the torque cable 321 can be used during deployment to support the sensor 106 in addition to the GWL 312. The torque cable 321 will hold the sensor 106 in place while the GWL 312 is retracted. This may enable various benefits, such as to test the anchoring of the sensor 106, provide the ability to interrogate the sensor 106 with no interference, final positioning of the sensor 106, and/or repositioning of the sensor 106 when the sensor 106 is still attached to the torque cable 321.

When the physician or practitioner is ready to release the sensor 106, the torque cable 321 can be rotated counterclockwise and the threaded fastener 323 unscrews from the hole 325 in the sensor 106 while the sensor 106 is held in the vessel with the distal loop 324. In some embodiments, the torque cable 321 can move in the proximal direction 108 and/or the distal direction 110 independently of the torque cable lumen 311 to adjust the position of the sensor 106. If, in some cases, the torque cable 321 is turned in the clockwise direction, or opposite the direction used to separate the threaded fastener 323 from the sensor 106, the torque cable can be used to radially position the sensor 106 within the vessel.

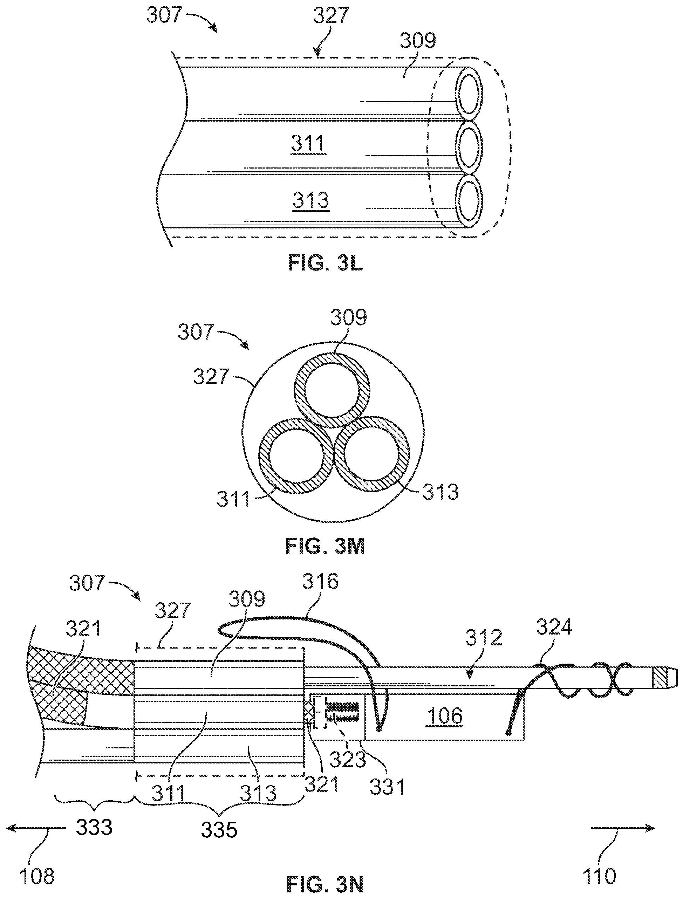

Figure 3L shows a portion of the tri-lumen catheter 307 wherein the GWL lumen 309, torque cable lumen 311, and pressure lumen 313 are linearly aligned in accordance with embodiments herein. Heat shrink 327 can be applied around the lumens 309, 311, and 313 to keep the lumens in the desired alignment with respect to each other. In some cases, the heat shrink material can extend the full length or nearly the full length of the tri-lumen cable except for the hubs 315, 317, and 319. Figure 3M shows an end view of the tri-lumen catheter 307 having the GWL lumen 309, torque cable lumen 311, and pressure lumen 313 arranged triangularly with respect to each other and encased in heat shrink 327 in accordance with embodiments herein. It should be understood that other configurations may be used, including one or more additional lumen, while still maintaining the relationship of the GWL 312 to the sensor 106 and the torque cable 321 to the sensor 106. Optionally, it should be understood that instead of independent lumens as described, a single extrusion may be created with the same geometry.

Figure 3N is another view of the tri-lumen catheter 307 that shows the sensor 106 in accordance with embodiments herein. The threaded fastener 323 is shown protruding from the distal end of the torque cable 321 into a nut 331 positioned at the proximal end of the sensor 106. An outline of upper and lower portions of the heat shrink 327 is shown for reference.

Additionally, similar to the discussion above with respect to Figure 3B, one or more of the GWL lumen 309, torque cable lumen 311, and pressure lumen 313 can have variable stiffness along their lengths. In this example, a proximal portion 333, such as from the hubs 315, 317, 319, extending approximately 1 - 30 mm distal to the delivery system (e.g., proximal end of sensor 106), or a proximal portion 333 extending a greater portion of the GWL 312, can be reinforced or stiffer than a remaining distal portion 335. Reinforcement can be similar to that discussed above. In some embodiments, the proximal portion 333 can have a value of approximately 63 D or more, while the distal portion 335 can have a value of approximately 55 D or less. The stiffer proximal portion can provide the benefit of improved pushing and control of the catheter 307 through the vessel.

### Distal Loop Interlace

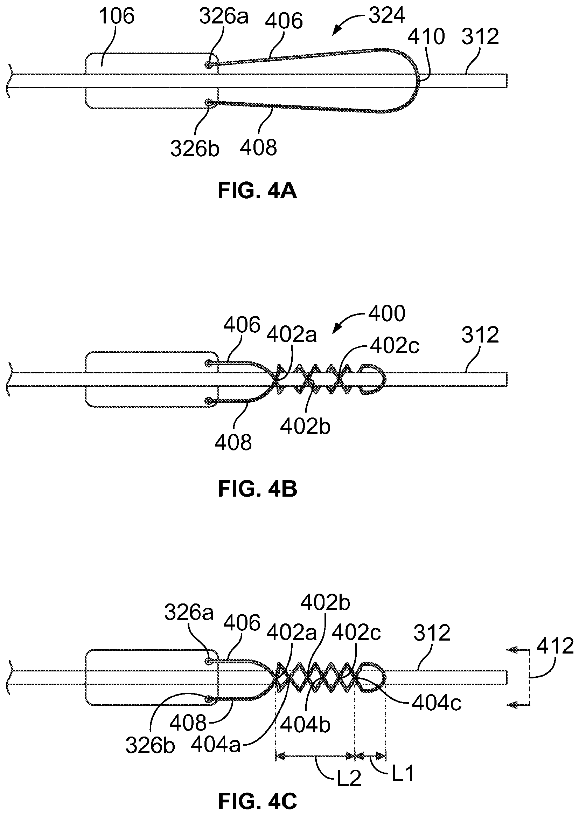

Figures 4A-4C illustrate top-down views of the interlacing of the distal loop 324 on the GWL 312 in accordance with embodiments herein. Figure 4A shows the GWL 312 and sensor 106 in parallel with each other. The distal loop 324 is shown and discussed in portions or halves for reference only; it should be understood that the distal loop 324 is one contiguous piece of material and that the distal loop portions 406, 408 of the distal loop 324 may be the same length or different lengths. Further, although some embodiments herein may be discussed as having a top or bottom in orientation, it should be understood that the embodiments are not limited to a particular orientation. The distal loop portion 406 is attached to the sensor 106 at the attachment point 326a and the distal loop portion 408 is attached to the sensor 106 at the attachment point 326b. The distal loop 324 is shown as being on an "out of page" side, or in front of, the GWL 312 at position 410.

Referring to Figure 4B, this figure shows the weave or interlace pattern 400 with the GWL 312 in place, and thus illustrates portions of the interlace pattern in the "out of page" direction. In Figure 4C, the GWL 312 is transparent in the section corresponding to the interlace pattern to show the interlace pattern in both the "out of page" direction (e.g., in front of the GWL 312) and "into page" direction (e.g., in back of or behind the GWL 312).

In Figure 4B, when the distal loop portions 406 and 408 cross in the out of page direction at cross points 402a, 402b, 402c (e.g., along a first or top side of the GWL 312), the distal loop portion 408 is always in front of the distal loop portion 406. Therefore, the distal loop portion 408 is out of page compared to the distal loop portion 406. In other words, the distal loop portion 408 is outside of the distal loop portion 406, or the distal loop portion 406 is between the distal loop portion 408 and the outer surface of the GWL 312.

Referring specifically to Figure 4C, cross points 404a, 404b, 404c located on the "into page" side of the GWL 312 (e.g., along a second or bottom side of the GWL 312) are shown together with the cross points 402a, 402b, 402c of the "out of page" side of the GWL 312. The GWL 312 is transparent in the area corresponding to the interlaced pattern in Figure 4C to clearly show the cross points 404, but it should be understood that the orientation of the assemblies of Figures 4B and 4C are the same. Therefore, in some embodiments the cross points 402 and 404 can be located on different sides of the GWL 312, and in other embodiments, the cross points 402 and 404 can be located on substantially opposite sides of the GWL 312. It should be understood that the GWL 312 has, in some embodiments, a substantially cylindrical shape. Therefore, the terms "sides", when used with respect to the GWL 312, do not refer to a flattened side but a reference position with respect to each other. For example, one "side" could be a top side (out of page), such as the portion of the GWL 312 visible in Figure 4B, while another "side" could be a bottom side (into page) of the GWL 312 that is not visible in Figure 4B.

Again, one of the loop portions, in this example the distal loop portion 408, is always in front of the distal loop portion 406, or in the "out of page" direction. In other words, on the "into page" side or back side of the GWL 312, one of the distal loop portions (e.g., distal loop portion 406) is always on top or on an outside of the cross points 404, while on the "out of page" side of the GWL 312, the other distal loop portion (e.g., distal loop portion 408) is always on top or on the outside of the cross points 402. This allows each side of the distal loop 324, the distal loop portions 406 and 408, to "pop" into place without twisting rotationally (e.g., around each other) when the GWL 312 is removed.

Therefore, when viewed as shown in Figures 4b and 4C, the distal loop portion 406 can be always in front of, or in the "out of page" direction, of the distal loop portion 408. However, if the distal loop portion that is "out of page" varies, such that the distal loop portion 406 is "out of page" at one or more cross points 402, 404 and the distal loop portion 408 is also "out of page" at one or more cross points 402, 404, the distal loop 324 of the sensor 106 may not deploy correctly when the GWL 312 is removed.

Although six cross points 402, 404 are shown in Figures 4B and 4C, the interlace pattern 400 is not bounded by the number of cross points 402, 404. A minimum of one cross point 402, 404 is needed. The number of cross points 402, 404 may be dependent on a length of the distal loop 324, a circumference of the GWL 312, etc. For example, the cross points 402, 404 provide enough friction such that when the distal loop 324 passes through an introducer valve or hemostasis valve (not shown), the interlace pattern 400 is maintained and thus deploys properly to its oval or circular deployment shape rather than bunching up on itself. In some embodiments, the number of cross points 402, 404 of the interlace pattern 400 can be selected to keep the profile of the interlace pattern 400 low enough and tight (or secure) enough to be passed through the introducer or hemostasis valve without causing the distal loop 324 to bunch up around the GWL 312 and/or fold back around the sensor 106.

Therefore, although the interlace pattern 400 of the distal loop 324 is illustrated as loosely positioned around the outer surface of the GWL 312 for ease of description, at least portions of the interlace pattern 400 may be flush and/or in contact with an outer surface of the GWL 312 while still providing a level of friction that allows the GWL to be easily removable. For example, in some cases a level of friction force that inhibits the pulling force of the GWL 312 is not desirable. In some embodiments, the interlace pattern 400 may not have a uniform tightness along the GWL 312, wherein a tip portion L1 of the interlace pattern 400 may be looser than a body portion L2 of the interlace pattern 400.

Some advantages of interlacing the distal loop 324 to the GWL 312 include keeping the distal loop 324 at a low profile for easy navigation and preventing the distal loop 324 from inverting proximally due to insertion/navigation forces through the body. The specific weave pattern 400 prevents any twisting of each side of the distal loop 324, which allows for long term loop form stability and prevention of tangling of the distal loop 324 once released.

Referring again to Figure 4C, a longitudinal plane 412 can be considered to bisect the cylindrical GWL 312. The cross points 402 are forward (e.g., to a left side if viewed from an end of the GWL 312, to a first side, etc.) of the longitudinal plane 412 while the cross points 404 are behind (e.g., to a right side if viewed from the end of the GWL 312, to a second side, etc.) of the longitudinal plane 412. The portion of the distal loop 324 that extends from, for example, the attachment point 326b (e.g., the distal loop portion 408) will be further from the longitudinal plane 412 at the cross points 402 than the portion of the distal loop 324 that extends from the attachment point 326a (e.g., the distal loop portion 406). The portion of the distal loop 324 that extends from the attachment point 326b (e.g., the distal loop portion 408) will be closer to the longitudinal plane 412 at the cross points 404 than the portion of the distal loop 324 that extends from the attachment point 326a (e.g., the distal loop portion 406).

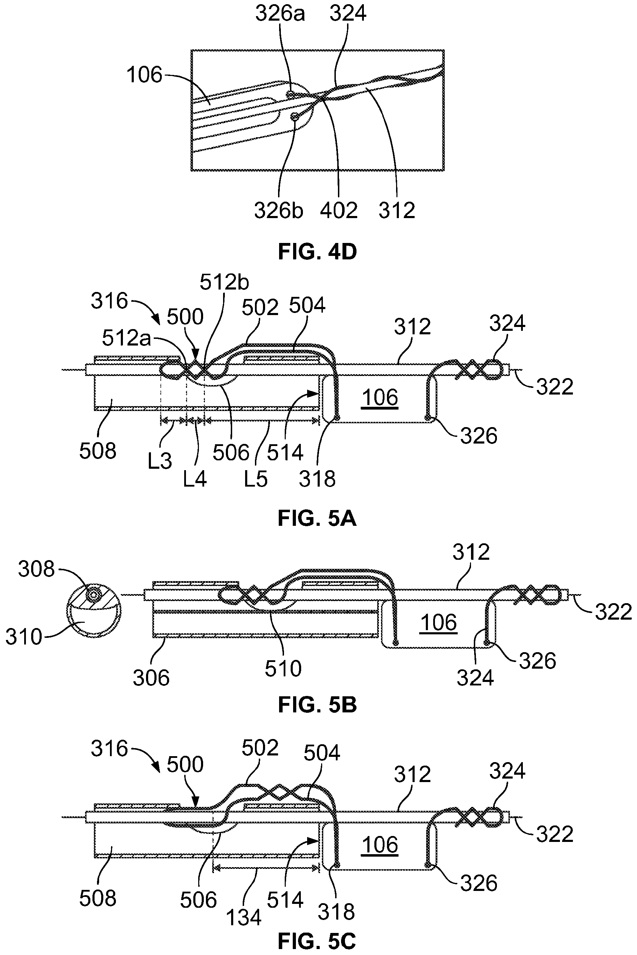

Figure 4D illustrates the sensor 106 and distal loop 324 secured to the sensor 106 at attachment points 326a, 326b in accordance with embodiments herein. The cross point 402 closest to the sensor 106 is positioned on a side of the GWL 312 that is furthest from the sensor 106, although the embodiments herein are not so limited.

In other embodiments, the distal loop 324 can be twisted or wrapped around the GWL 312 in other patterns. For example, the distal loop portions 406 and 408 may be positioned next to, near each other, not touching, etc., and wrapped around the outer surface of the GWL 312 one or more times, such as without being interlaced. In further embodiments, the distal loop portions 406 and 408 can be twisted around each other one or more times before being wrapped one or more times around the outer surface of the GWL 312. In still further embodiments the distal loop portions 406, 408 can be twisted in the same direction, alternating which portion is on the outside of either side of the GWL 312. It should be understood that the twisting, wrapping, and/or interlacing can be accomplished in any order and in any combination. Advantages of twisting the distal loop include increased stiffness and/or stability to help retain the distal loop in the desired position.

### Proximal Loop Interlace

Figures 5A and 5B illustrate cross-sectional views of proximal loop interlacing in accordance with embodiments herein. Figure 5C illustrates another cross-sectional view of the proximal loop retained by the GWL 312 wherein portions of the proximal loop are twisted together or around each other in accordance with embodiments herein. Figures 5A and 5B show the GWL 312, guidewire 322, and distal loop 324 arranged in an interlace pattern to removably couple the distal loop 324 to the GWL 312. The distal loop 324 is attached to the sensor 106 at two attachment points 326 (one is shown). Figures 5A and 5C illustrate a single lumen catheter 508 while Figure 5B illustrates the multi-lumen catheter 306. It should be understood that the embodiments and variations disclosed herein can be accomplished using a single lumen catheter 508 or a multi-lumen catheter 306. If the single lumen catheter 508 is used, the GWL 312 may move within the single lumen and thus may be positioned closer to a center of the catheter 508 compared to the positioning with the multi-lumen catheter 306, wherein the GWL 312 and sensor 106 are closer to concentric.

In Figures 5A and 5B, the proximal loop 316 is arranged in an interlace pattern 500 around an outer surface of the GWL 312. The proximal loop 316 includes proximal loop portions 502 and 504, wherein proximal loop portion 504 is secured to the sensor 106 at attachment point 318. The attachment point securing the proximal loop portion 502 to the sensor 106 is not shown. The interlace pattern 500 can be accomplished as discussed in Figures 4B and 4C with respect to the distal loop 324, ensuring that when the proximal loop 316 is released, the proximal loop 316 will pop without twisting rotationally. Therefore, the interlace pattern 500 can have one or more cross points 512 as needed to wrap the proximal loop 316 around the GWL 312 while ensuring that the GWL 312 can be removed without unnecessary friction. The cross points 512a, 512b are shown on the "out of page" side.

Skive 506 provides space for the interlace pattern 500 of the proximal loop 316 to be held within the single lumen catheter 508. The skive 506, in some cases, can be larger or more extensive than the skive 314 of Figure 3A that accommodates the proximal loop 316 that is not arranged in an interlace pattern.

In some embodiments, as discussed above in Figure 4C, a tip portion L3 of the interlace pattern 500 may be looser than a body portion L4 of the interlace pattern 500. The number of cross points 512 can be determined depending upon one or more of distance of the skive 506 from the distal end 514 of the catheter 508 and the sensor 106, length of the proximal loop 316, height profile desired within an extending portion L5 of the proximal loop 316, desired tightness/friction with respect to the GWL 312, etc.

Figure 5B is similar to Figure 5A, except that the multi-lumen catheter 306 is used. Again, a larger skive 510 can be formed by removing catheter material. In this example, the skive 510 may extend into the second lumen 310.

Figure 5C illustrates an embodiment wherein the proximal loop portions 502 and 504 are not interlaced around the GWL 312 within the skive 506 as discussed previously in Figure 5A. In some embodiments, sections of the proximal loop, such as the proximal loop portions 502, 504 and/or lengths of one or both of the proximal loop portions 502, 502 extending within area 134, can be twisted around each other or themselves. Advantages of twisting the proximal loop include increased stiffness and/or stability to help retain the proximal loop in the desired position during deployment.

Referring also to Figures 3A-3C, the material of the proximal loop 316 can be stored within inner area(s) of the multi-lumen or single catheter 306, 508, within the skive 314, 506, 510, or within a combination of the catheter 306, 508 and skive 314, 506, 510. Accordingly, the skives 314, 506, 510 can be sized as needed, accommodating all or a portion of the interlaced proximal loop 316.

### Pressure Sheath

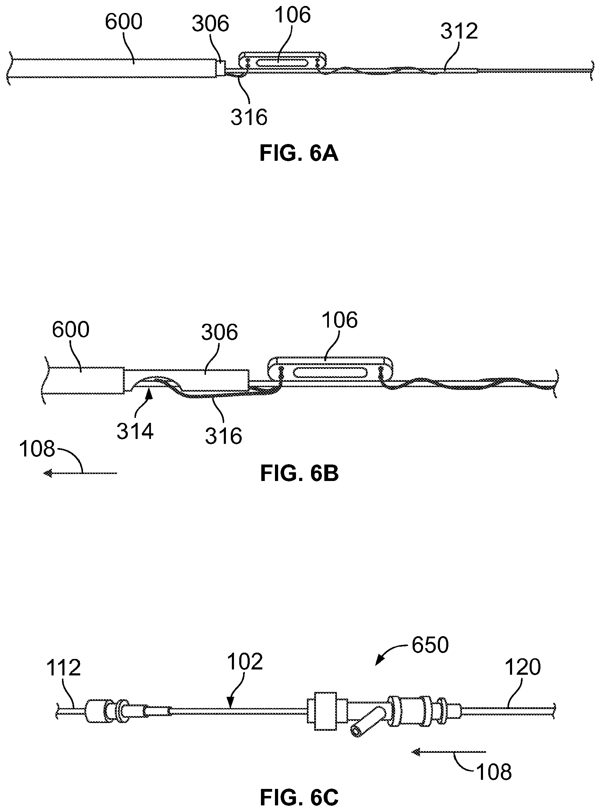

Figures 6A and 6B illustrate a pressure sheath 600 positioned over at least a portion of the catheter 306 in accordance with embodiments herein. Figure 6B illustrates the pressure sheath 600 retracted in the proximal direction 108 such that at least a portion of the skive 314 is shown.

When the pressure sheath 600 is positioned over the skive 314 (Figure 6A), the pressure sheath 600 acts as an additional locking mechanism to securely lock the proximal loop 316 with the GWL 312. Portions of the proximal loop 316 extend between the catheter 306 and the pressure sheath 600, and portions of the proximal loop 316 are positioned or held within the skive 314 and/or multi-lumen catheter 306.

In some embodiments the pressure sheath 600 can be used to take pressures before and/or after release of the sensor 106 without an added step of removing the delivery sheath (e.g., introducer sheath) and reinserting a pressure catheter. Instead, pressures can be taken through the pressure sheath 600. In some cases, the multi-lumen catheter 306 is removed while the pressure sheath 600 remains in place, and then pressures are taken through the pressure sheath 600. By way of example only, the pressure sheath 600 can be a column of fluid, wherein blood of the patient applies pressure at the end of the body. A pressure transducer (not shown) can be connected to the pressure sheath 600 outside of the body to measure the pressure of the column of fluid.

In some embodiments, a lumen of sufficient diameter within the multi-lumen catheter 306 could contain an available channel (e.g., second lumen 310 of Figure 3A) for measuring pressures, such as PAP. In some cases, taking the pressure through an available lumen may eliminate the need for the pressure sheath 600. Thus, in some embodiments, a locking sleeve (not shown) can be used instead of a pressure sheath 600, wherein the locking sleeve secures the proximal loop 316 (e.g., trap and lock) within the skive 314 and to the catheter 306.

Further, the pressure sheath 600 can be used with proximal interleaving/interlacing as discussed in Figures 5A and 5B or with the proximal loop 316 positioned between the GWL 312 and catheter 306/skive 314 as shown in Figure 3A.

Figure 6C illustrates translational components 650 on the proximal end of the catheter 306 that allow for translational control of the sensor delivery system 300. For example, translational components 650 such as various y-connectors and/or luers can control the GWL 312, catheter 306, pressure sheath 600, other outer sheaths/jackets/inner shafts/components, etc. These components and/or others not shown (e.g., 2e lock, push squeeze lock, etc.) lock the sensor 106 in place until the practitioner is ready to proceed with the release. There must be enough distance along the catheter 306 between other components and the pressure sheath 600 to allow the pressure sheath 600 to be pulled far enough back to uncover the skive 314 (as shown in Figure 6B) and allow the release of the proximal loop 316 from the catheter 306 when the GWL 312 is pulled a sufficient distance in the proximal direction 108.

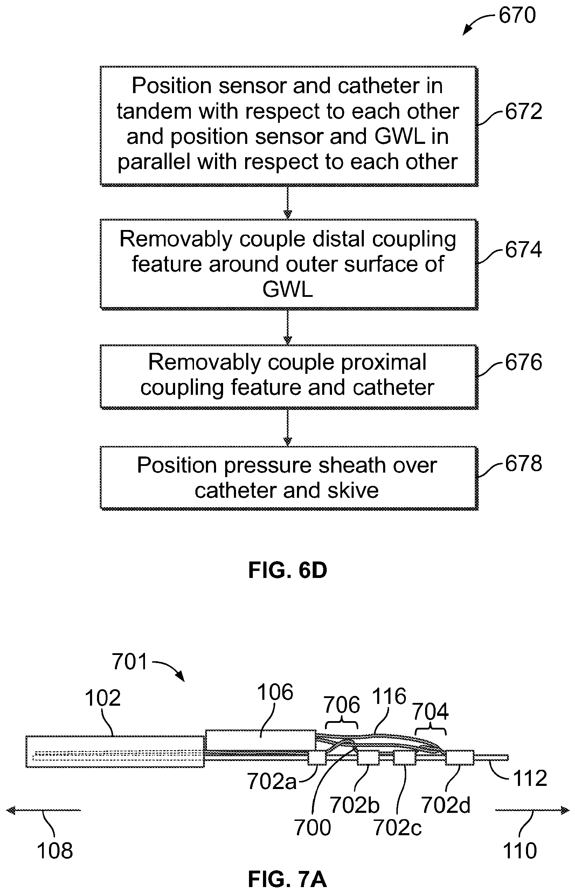

Figure 6D illustrates a method 670 for forming a tandem sensor delivery system 100 that can deliver an intracorporeal sensor 106 in accordance with embodiments herein. It should be understood that the order of the steps may change and are not limited to those discussed herein.

At 672, the sensor 106 and catheter 306 are positioned in tandem and the sensor 106 and the GWL 312 are positioned in parallel with each other. For example, the proximal end 328 of the sensor 106 can be located distal with respect to the distal end 334 of the catheter 306.

At 674, the distal loop 324 (e.g., distal coupling feature) of the sensor 106 can be removably coupled to the GWL 312 by arranging the distal loop 324 around an outer surface of the GWL 312, such as in the interlace pattern 400 discussed in Figures 4B and 4C. The GWL 312 extends from the distal end 334 of the catheter 306 and accommodates the interlace pattern 400 distally with respect to the distal end 330 of the sensor 106.

At 676, the sensor 106 is removably coupled to the catheter 306. In some embodiments, the proximal loop 316 (e.g., proximal coupling feature) of the sensor 106 can be arranged around the outer surface of the GWL 312, such as in the interlace pattern 500 discussed in Figures 5A and 5B. The interlaced portion of the proximal loop 316 can be positioned within the skive 314 and/or catheter 306. In other embodiments, the proximal coupling feature is retained within the skive 314 and/or catheter 306. For example, the proximal loop 316 can be retained between the GWL 312 and the catheter 306 and/or skive 314 as shown in Figure 3A. Additional material of the proximal loop 316 can be stowed within the skive 314 and/or catheter 306.In other embodiments, a torque cable 321 can be used to couple a threaded fastened 323 with the sensor 106, as discussed herein. In still further embodiments, a floss, ring, paddles, or other coupling feature can extend from the catheter and/or from within the catheter and be retained by a retention feature (e.g., loop, hole, ring, ball) in the sensor 106 and/or interconnected with a retention feature, as discussed herein.

The interlocking embodiments of 674 and 676 may require access to a proximal end (not shown) of the GWL 312 and the distal tip 320 of the GWL 312, as well as manipulation of the position of the GWL 312 along the catheter 306.

At 678, in some embodiments, the pressure sheath 600 or other sleeve can be positioned over the catheter 306 and the skive 314, providing the benefit of taking pressures with the pressure sheath 600 as well as more securely locking the sensor 106 and catheter 306 to each other. The assembly provides an additional advantage wherein the practitioner does not need to handle the sensor 106 separately from the catheter 306 or interconnect the sensor 106 with the catheter 306. In other embodiments, a lumen in a multi-lumen catheter (e.g., two lumen, three lumen, etc.) can be used to take pressure readings, which can eliminate the need for the pressure sheath 600.

### Rubber Bumpers and Floss

The tandem delivery, wherein the sensor 106 and the catheter 306 are positioned in tandem with respect to each other and the sensor 106 and the GWL 312 are in parallel with each other, can be utilized with release mechanisms other than, or in combination with, the proximal loop interlocking and the proximal and distal loop interlacing discussed herein.

Figure 7A illustrates a cross-sectional view of another sensor delivery system 107 having the sensor 106 and catheter 102 positioned in tandem, wherein the distal loop 116 is secured to the GWL 112 with floss 700 and rings or rubber bumpers 702 in accordance with embodiments herein. For example, the floss 700 can be a biocompatible floss, and may include one or material such as suture, metal, polymer, etc. The proximal loop is captured within the catheter and thus is not shown in this view. A plurality of bumpers 702a, 702b, 702c, 702d (e.g., chronoprene, tecothane, polyurethane, silicon, etc.) secure the floss 700 to the GWL 112. The floss 700 is looped at least once around or through the distal loop 116, such that at least at one point the distal loop 116 is held between the floss 700 and the GWL 112. In this example, within area 704, the floss 700 extends from a distal end of bumper 702c and captures the distal loop 116 between the floss 700 and the GWL 112 before extending under a proximal end of the bumper 702d. Further, within area 706, the floss 700 extends from a distal end of bumper 702a and captures the distal loop 116 between the floss 700 and the GWL 112 before extending under a proximal end of the bumper 702b. In other embodiments the distal loop 116 can be captured in one area or in three or more areas.

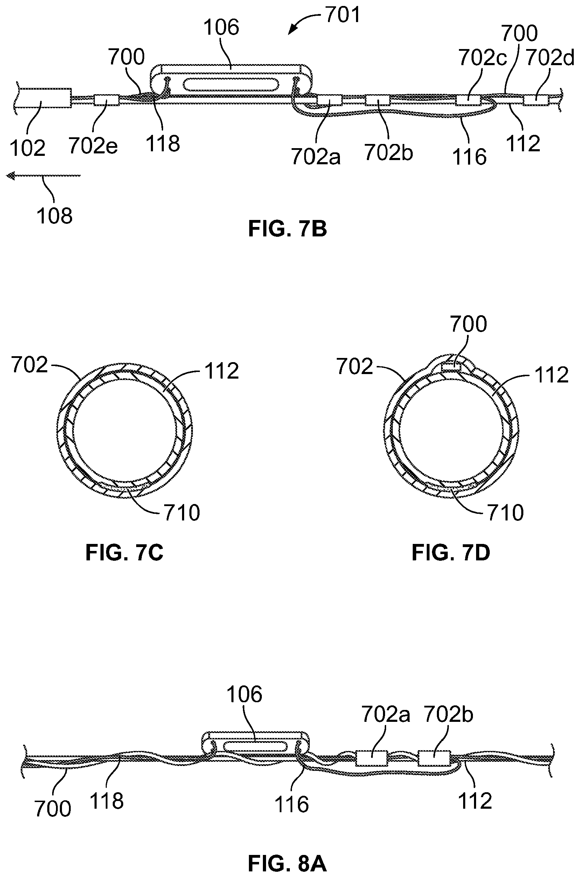

Figure 7B illustrates another view of the delivery system 107 of Figure 7A in accordance with embodiments herein. As discussed in Figure 7A, the distal loop 116 can be captured in one or more areas by the floss 700 that is held to the GWL 112 by one or more bumpers 702. The catheter 102 has been pulled in the proximal direction 108, showing that the same floss 700 can secure the proximal loop 118 in the same manner to the GWL112 using one or more bumpers 702e (one is indicated).

To release the sensor 106 from the catheter 102, the floss 700 can be pulled by the practitioner in the proximal direction 108. The distal loop 116 is released from the delivery system 701 when the floss 700 has been pulled in the proximal direction 108 and releases the distal loop 116 within the attachment area 706 closest to the sensor 106. The proximal loop 118 is similarly released. Figure 7C shows a cross-sectional view of the bumper 702 encircling the GWL 112 and Figure 7D includes the floss 700 held between the bumper 702 and the GWL 112 in accordance with embodiments herein. The bumper 702 is thermal or adhesively attached on one point 710 (e.g., tack weld, etc.) and the floss 700 will go through the loose sections (e.g., untacked sections). Due to the stretch caused by the size of the floss 700, the bumper 702 will experience radial compression to the GWL 112 which holds the floss 700 in place.

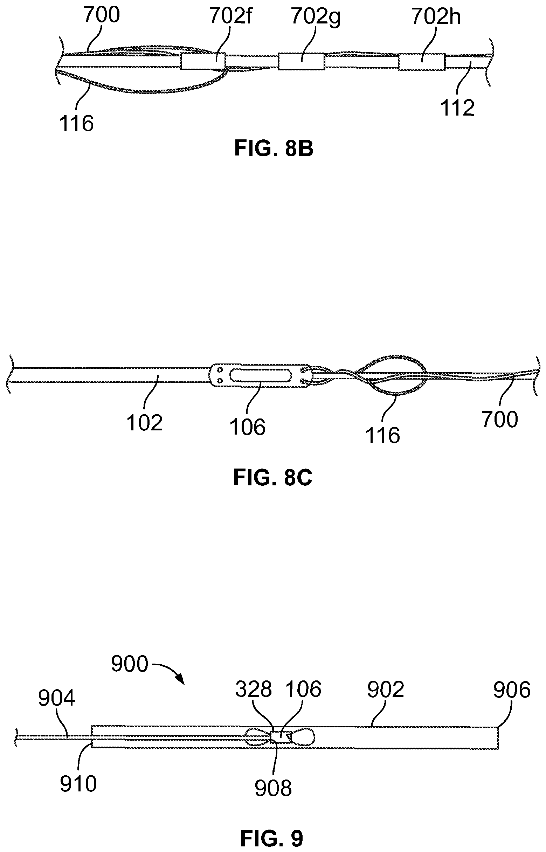

Figures 8A-8C illustrate views of the sensor 106 and catheter 102 positioned in tandem, wherein the distal loop 116 is secured to the GWL 112 with floss 700 and bumpers 702 in accordance with embodiments herein. The floss 700 can be wrapped or twisted around the GWL 112 and can also be wrapped or twisted around the proximal loop 118. In other embodiments, the profile associated with the floss 700 and/or distal and proximal loops 116, 118 can be minimized and the floss 700 can be securely held in place when being loaded into the catheter 102 and/or other sheaths. Referring to Figure 8B, the bumper 702f secures the distal loop 116 and the floss 700 to the GWL 112, while the bumpers 702g, 702h secure the floss 700 to the GWL 112. In some cases, more or less bumpers 702 can be used.

In some embodiments the floss 700 can be a full floss, wherein the floss is a full-length tether connection. The practitioner pulls the floss 700 at least as far as needed to release the distal loop 116 and the proximal loop 118. Once the GWL 112 and the floss 700 are proximal with respect to the distal loop 116, the distal loop 116 will deploy to its predetermined shape and engage the walls of the vessel. Similarly, the proximal loop 118 will deploy when released from the floss 700 and the catheter 102 and GWL 112 are proximal with respect to the proximal loop 118.

In other embodiments, the floss 700 can be a hybrid floss, wherein the practitioner pulls the floss and/or actuates a cutter to cut the floss which releases the proximal loop 118. The distance the floss 700 needs to be pulled is much shorter compared with the full floss. The operation of the hybrid floss and cutter are described further below.

### Sheathed Delivery System Overview

In coronary cases for various interventions, the practitioner inserts a guide catheter that has a dilator on the distal end that transitions down to a guidewire. This process places a hollow tube or sheath extending from the proximal end (e.g., the practitioner) to the distal end (e.g., location where implantable device is to be positioned in the vessel). The insertion of the delivery sheath can be as shown and discussed above in Figures 2A and 2B. In the below sheathed delivery systems, once the sheath is in place within the patient the sensor can be advanced without a guidewire.

Figure 9 illustrates a sheathed delivery system 900 to deliver the sensor 106 in accordance with embodiments herein. A push rod 904 extends proximally beyond a proximal end 910 of delivery sheath 902. The push rod 904 pushes the sensor 106 through the delivery sheath 902 to and beyond distal end 906 of the delivery sheath 902. Therefore, the push rod 904 pushes the sensor 106 the entire way to the deployment site. To control the location and position of the sensor 106, the proximal end 328 of the sensor 106 is removably attached to a distal end 908 of the push rod 904 with a coupling mechanism (e.g., a plurality of coupling mechanisms are discussed further below). For example, a plurality of release mechanisms are configured to uncouple the push rod 904 from attachment features of the sensor 106.

The delivery sheath 902 is a shaft construction that typically includes a liner, braid, and polymer jackets. The delivery sheath 902 passes through an introducer sheath over the guidewire and dilator (not shown). After placement, the dilator and guidewire can be removed from the delivery sheath 902 so it is ready for the sensor delivery.

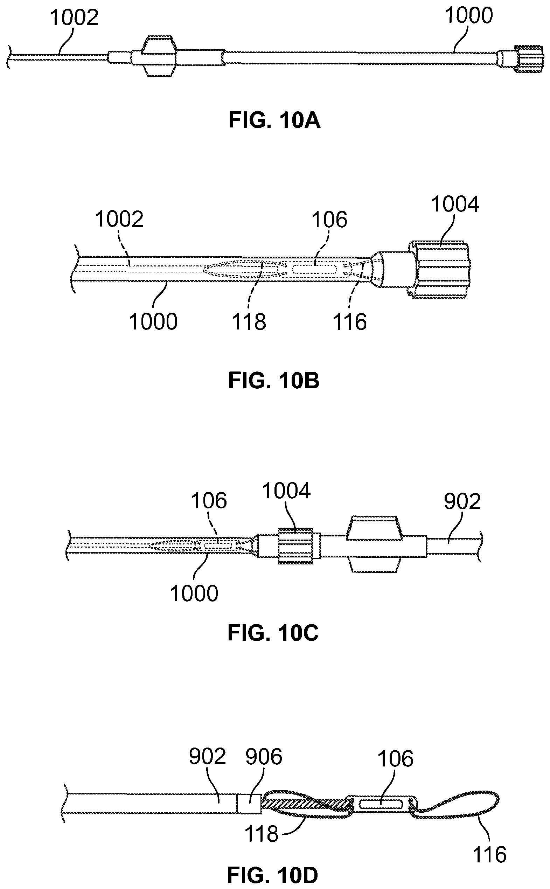

The sensor 106 and push rod 904 can be preloaded in a loader tube that connects directly to the delivery sheath 902. Figure 10A illustrates a loader tube 1000 attached to a threaded delivery cable 1002, and Figure 10B illustrates an image of the sensor 106 in the loader tube 1000 in accordance with embodiments herein. In some embodiments, the threaded delivery cable 1002 operates as the push rod 904. The loader tube 1000 has an attachment fitting 1004 on one end. The distal loop 116 and proximal loop 118 are on either end of the sensor 106.

Figure 10C illustrates the loader tube 1000, with the sensor 106 therewithin, attached to the delivery sheath 902, and Figure 10D illustrates the sensor 106 deployed from the delivery sheath 902 in accordance with embodiments herein. In Figure 10D both the distal loop 116 and the proximal loop 118 are free of the distal end 906 and have expanded. When deployed within a patient's vessel, the distal and proximal loops 116, 118 can engage the vessel walls, holding the sensor 106 in place within the vessel.

Accordingly, the sensor 106 and the push rod 904 (e.g., threaded delivery cable 1002) can be preloaded in the loader tube 1000 that connects directly to the delivery sheath 902. The loader tube 1000 aids in keeping the distal and proximal loops 116, 118 (e.g., anchor loops) of the sensor 106 compressed and in the correct orientation for proper anchoring post-delivery. The diameter of the distal and proximal loops 116, 118 is larger than an inner diameter of the delivery sheath 902, and thus the distal and proximal loops 116, 118 will not enter the deliver sheath 902 without manipulation. Advantages of using the loader tube 1000 is that the loader tube 1000 eliminates this compression step for practitioners and minimizes contact with the sensor 106, as this step could be accomplished, for example, during an assembly process in advance of use by the physician.

Embodiments disclosed herein include features that allow alternate securement options, delivery techniques, and release mechanisms that can be used to deliver the sensor 106. Once the sensor 106 is advanced distally out of the delivery sheath 902, the sensor 106 is released, such as by disengaging the release mechanism of the push rod 904 from the attachment feature of the sensor 106. After release, the push rod 904 and release mechanism are removed. An advantage of the system is that the delivery sheath 902 remains proximal to the deployed sensor 106, enabling pressure readings to be taken without removing the delivery sheath 902 and reinserting a separate device, as has previously been standard practice.

An additional benefit of the sheathed delivery system 900 is that it can be delivered over any standard size guidewire the practitioner wishes to use. Currently, a 0.018 inch guidewire is used. The sheathed delivery system 900 can accommodate guidewires that are 0.018-0.035 inch in diameter without requiring the size of the introducer sheath to be changed (e.g., 12F).

### Hybrid Suture Floss Cut Release

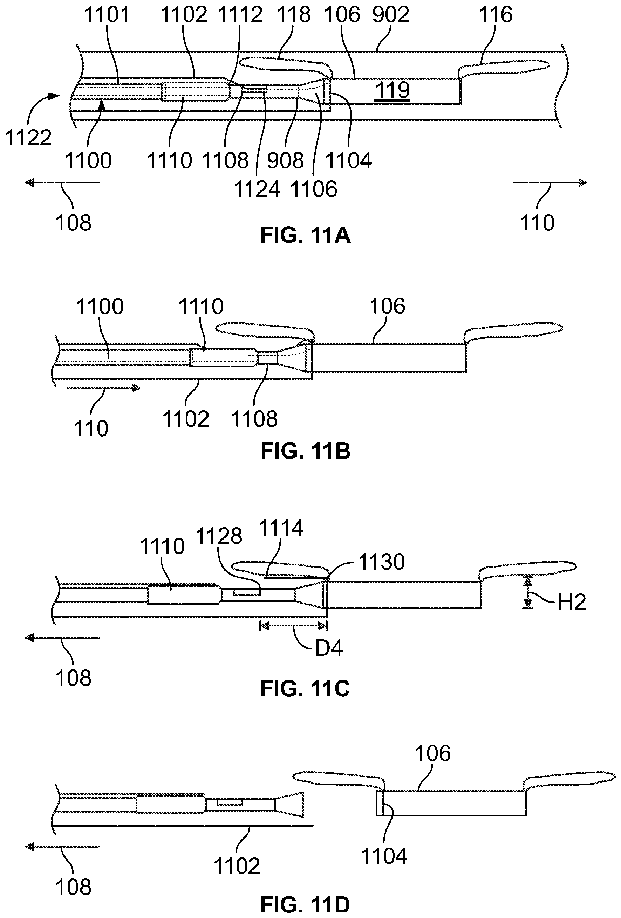

Figures 11A-11D illustrate a hybrid suture floss cut release in accordance with embodiments herein. Figure 11A illustrates a cross-sectional view of the sensor 106 and push rod 1100 within the delivery sheath 902 in accordance with embodiments here. A floss 1102 can be a full-length floss that runs from proximal of a proximal end 1122 of the push rod 1100 (e.g., accessible to the practitioner), inside the delivery sheath 902, through a loop or hole 1104 in the sensor 106, and back to the proximal end 1122 of the push rod 1100. Therefore, a cutter mechanism can sever the floss 1102 into two pieces, and the floss 1102 can be a release mechanism that is removably coupled to an attachment feature, e.g., loop or hole 1104, of the sensor 106. In this example, the hole 1104 is provided through the body 119 of the sensor 106A. Bumper 1106 (e.g., soft rubber bumper) can be fastened to the distal end 908 (e.g., generally or near the distal end) of the push rod 1100. The bumper 1106 can be positioned between the distal end 908 of the push rod 1100 and the sensor 106 and/or be provided around the release mechanism such that portions of the push rod 1100 and the release mechanism extend through the body of the bumper 1106. Although an exemplary cutter mechanism is described herein, it should be understood that other mechanisms may be used to sever the floss 1102.

In some embodiments the push rod 1100 is configured to be longer than the delivery sheath 902, such as a few centimeters longer depending upon a length of the sensor 106 and the distal and proximal loops 116, 118. In some cases, the push rod 1100 may extend a distance out the proximal end of the delivery sheath 902 before the push rod 1100 is pushed to advance the sensor 106. The extra length ensures that the sensor 106 will exit the delivery sheath 902.

In some embodiments, the push rod 1100 is a composite of a braided push shaft 1101 and one or more tubes or mandrels (e.g., hypotubes). The floss 1102 is threaded through a window tube 1108. In other embodiments the floss 1102 extends outside the cutter tube 1110 and into an opening 1124 in the window tube 1108. The cutter tube 1110 has a bevel 1112 that is configured to cut the floss 1102 when the bevel 1112 of the cutter tube 1110 and the opening 1124 of the window tube 1108 are in a predetermined relationship with each other.

Figure 11B illustrates a cross-sectional view wherein the sensor 106 is out of the delivery sheath 902 (not shown) and is ready to be released in accordance with embodiments herein. For example, the push rod 1100 is pushed in the distal direction 110. The cutter tube 1110 is advanced in the distal direction 110 over at least a portion of the window tube 1108, and the bevel 1112 cuts/shears the floss 1102 extending from a hollow shaft of the window tube 1108 through the opening 1124.

Figure 11C illustrates a cross-sectional view wherein the cutter tube 1110 has been pulled in the proximal direction 108 and a severed end 1114 of the floss 1102 is indicated in accordance with embodiments herein. Figure 11D illustrates a cross-sectional view wherein the floss 1102 has been further pulled in the proximal direction 108 in accordance with embodiments herein. The floss 1102 has been pulled at least a distance to release the floss 1102 from the hole 1104 in the sensor 106. Advantageously, the pull of the floss 1102 can be short to release the sensor 106, such as a distance D4 between a proximal window edge 1128 to a hole 1130 in the sensor 106 that the floss 1102 extends into, plus a height H2 of the sensor 106. In some embodiments the pull of the floss 1102 can be an inch, two inches, etc. Further, in other embodiments, the distance the floss 1102 needs to be pulled can be based on the floss 1102 extending through a ring attached to the sensor 106 or other attachment mechanism as discussed further below. There is no need to pull the floss 1102 fully through the delivery sheath 902.

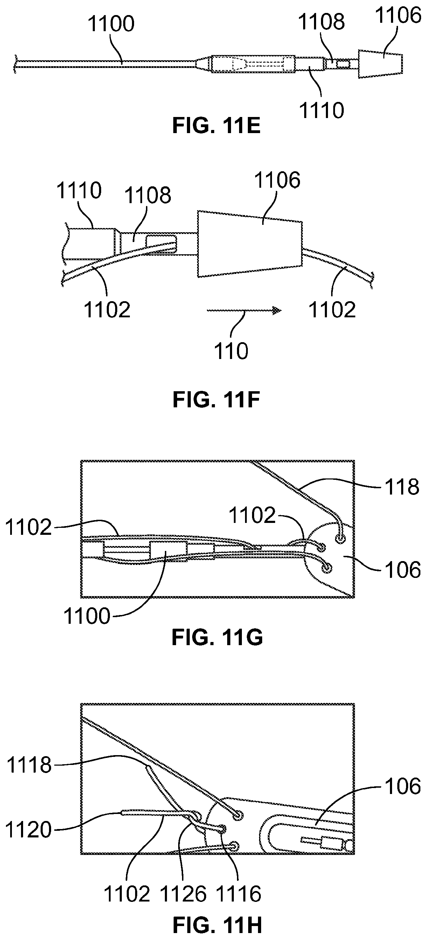

Figure 11E illustrates a view of the push rod 1100, window tube 1108, and bumper 1106, while Figure 11F illustrates another view of the window tube 1108 and bumper 1106, as well as the floss 1102 in accordance with embodiments herein. The floss 1102 can extend through a hole in the bumper 1106, and/or the bumper 1106 can have a flared tube-shape. The shape of the bumper 1106 is depicted as tapering in the distal direction 110; however, the shape of the bumper 1106 can vary. For example, the bumper 1106 can taper in the proximal direction 108, be different lengths, diameters, etc., and may be sized and/or shaped in accordance with a size and/or shape of the sensor 106.

Figures 11G and 11H illustrate an alternative embodiment that can sever the floss attachment to the sensor 106 without requiring the full length of floss 1102 to be pulled to release the sensor 106 in accordance with embodiments herein. In Figure 11G the push rod 1100 and sensor 106 are shown. The push rod 1100 includes the window tube 1108 and cutter tube 1110 but may not include the bumper 1106. Figure 11H illustrates that the floss 1102 is securely attached to the sensor 106, such as with a knot 1126 and extending through a hole 1116 in the sensor 106. The cutter tube 1110 has been actuated, cutting the floss 1102 in two positions indicated as severed ends 1118 and 1120. The sensor 106 is free of the delivery device after the floss 1102 has been severed. In other embodiments, the floss 1102 can have a single portion extending through the delivery device, wherein only one severed end 1118, 1120 is cut by the cutter tube 1110.

The hybrid suture floss cut release can also be used with the sensor 106 and the GWL 312, and/or in the tri-lumen catheter 307 configuration, such as for extra stability and to improve positioning/repositioning. A further advantage of combining the push rod 1100 and cutter assembly in the tandem configuration as discussed below is the smaller overall profile (e.g., diameter) of the assembly. Additionally, the assemblies can be used to deliver the sensor 106 without an additional delivery sheath ,e.g., such as delivery sheath 902 of Figure 11A, further decreasing diameter and complexity.

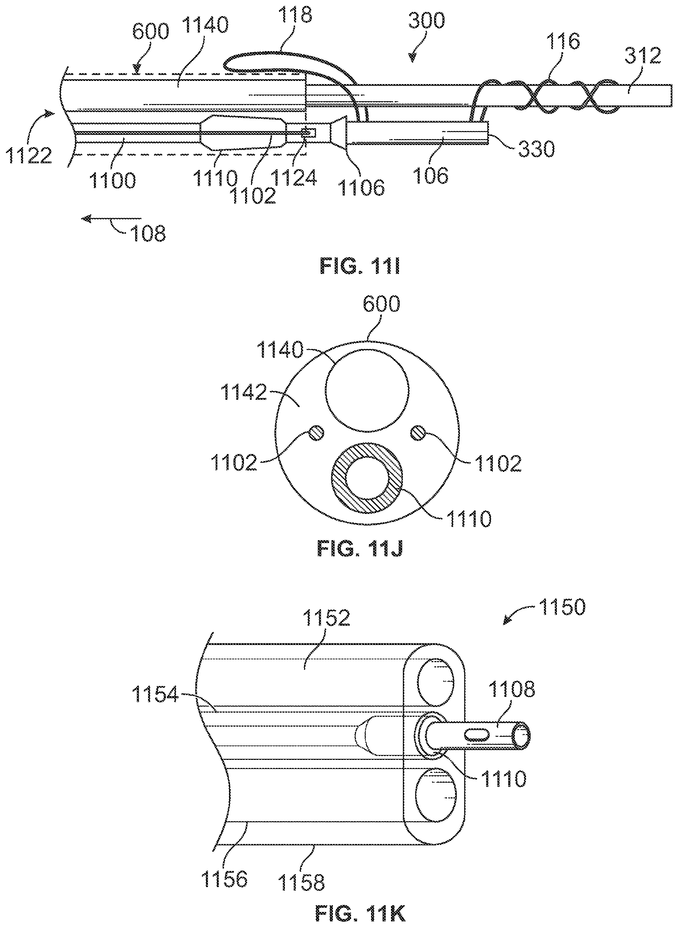

Figure 11I illustrates a sensor delivery system 300 having a cross-sectional view of a lumen 1140 held in parallel with the push rod 1100 and cutter, and positioned in tandem with the sensor 106 in accordance with embodiments herein. The lumen 1140 and push rod 1100 are held within a pressure sheath or jacket, such as pressure sheath 600. The pressure sheath 600 can, in some embodiments such as in Figure 11I, be referred to as a delivery catheter. The pressure sheath 600 can be used to extract pressure readings from the anatomy. The pressure sheath 600 extends from a proximal end 1122 of the assembly can extend nearly up to the sensor 106 or the distal end of the lumen 1140 and/or the distal end of the push rod 1100/cutter assembly. For example, the pressure sheath 600 can be shorter than the lumen 1140 and the push rod 1100/cutter assembly so that the pressure sheath 600 can be advanced or retracted to facilitate pressure readings after the sensor 106 is released.

The GWL 312 extends through the lumen 1140 and can contain a guidewire (not shown). The proximal loop 118 can be free-floating or unrestrained. In other embodiments, the proximal loop 118 can be retained inside the pressure sheath 600, inside the lumen 1140, and/or wrapped/interleaved around an outside surface of the GWL lumen 312 as discussed herein.

The GWL 312 extends beyond the distal end 330 of the sensor 106. The distal loop 116 is interleaved, wrapped around, etc., the GWL 312 as discussed further herein. The GWL 312 can be pulled in the proximal direction 108 to release the distal loop 116. The pressure sheath 600 can be pulled in the proximal direction 108 to release the proximal loop 118, if needed. Once the distal loop 116 is released, the floss 1102 can be pulled as discussed herein to release the sensor 106.

Figure 11J shows a cross-section of the pressure sheath 600, the lumen 1140 and the cutter tube 1110 in accordance with embodiments herein. The floss 1102 is shown in two places as, in some embodiments, both ends of the floss 1102 can extend to the proximal end 1122 of the system 300. Open area 1142 provides open space for measuring pressure levels.

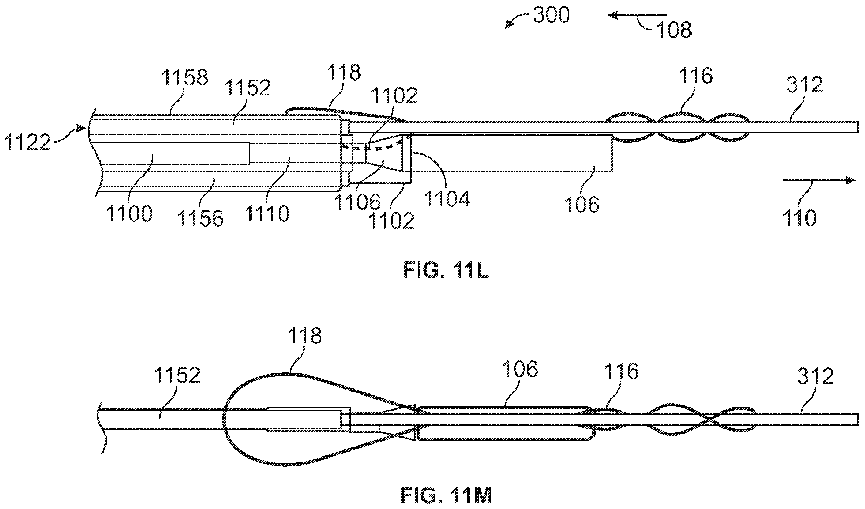

Figure 11K is a view of a portion of a tri-lumen catheter 1150 and Figure 11L illustrates a cross-sectional view of a sensor delivery system 300 that utilizes the tri-lumen catheter 1150 to deliver the sensor 106 within the body in accordance with embodiments herein. The tri-lumen catheter 1150 can generally be referred to as a delivery catheter. Figures 11K and 11L will be discussed together. The tri-lumen catheter 1150 is similar to the tri-lumen catheter 307 of Figure 3K, except that the torque cable 321 has been replaced with a floss and cutter assembly (e.g., cutter mechanism). The tri-lumen catheter 1150 includes three lumens, a first lumen referred to herein as a GWL lumen 1152 that accepts the GWL 312, a second lumen referred to herein as a cutter lumen 1154 that accepts a push rod 1100 and cutter tube 1110, and a third lumen referred to herein as a pressure lumen 1156 that facilitates measuring pressure. Located at a proximal terminal end (not shown) is a guidewire lumen hub, a cutter hub, and a pressure lumen hub. Heat shrink 1158 or other known bio-compatible material can be applied around the lumens 1152, 1154, and 1156 to hold the lumens together and to keep the lumens in the desired alignment with respect to each other (e.g., aligned in a single column or row, arranged triangularly) and to supply a smooth surface. In some cases, the heat shrink material can extend the full length or nearly the full length of the tri-lumen catheter 1150 except for the hubs. In some cases, the heat shrink material can be made of lubricious materials such as FEP, or PTFE.

Although not shown, the distal end of one or more of the GWL lumen 1152, pressure lumen 1156, and GWL 312 can have marker band (see markerbands 394 for Figure 3K for reference).

The GWL 312 extends from the GWL lumen 1152 in the distal direction 110. The distal loop 324 of the sensor 106 can be twisted, interlaced or otherwise wrapped around the GWL 312 as discussed herein. The proximal loop 118 of the sensor 106 may be free floating, or alternatively, can be captured in any manner discussed herein. In other embodiments, the sensor 106 has no proximal loop 118

In some cases, the floss 1102 extends within the cutter lumen 1156 proximate the push rod 110 and cutter tube 1110, enters the window tube 1108 (see Figure 11A) and extends through the bumper 1106 to enter the hole 1104 that runs through the body of the sensor 106. In other cases, the floss 1102 can extend through the pressure lumen 1156 or the cutter lumen 1154 to the proximal end 1122.

Figure 11M illustrates a top view of a portion of the sensor delivery system 300 in advance of releasing the distal loop 116 in accordance with embodiments herein. The heat shrink 1158 is not shown for clarity. The GWL 312 extends from GWL lumen 1152, and the distal loop 116 is interleaved around the outer surface of the GWL 312.

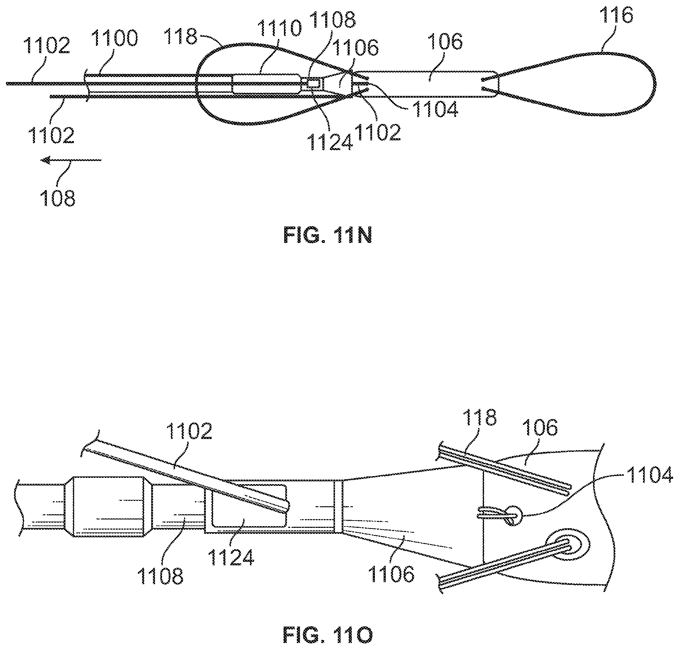

Figure 11N is a view showing the sensor 106, push rod 1100, cutter tube 1110, window tube 1108, and bumper 1106 of the sensor delivery system 300 of Figure 11K in accordance with embodiments herein. The GWL lumen 312 has been retracted in the proximal direction 108 and the distal loop 116 has been released. The floss 1102 extends along the push rod 1100 and the cutter tube 1110, enters the opening 1124 of the window tube, exits the bumper 1106 and enters the hole 1104 in the sensor 106. The floss 1102 exits the hole 1104 in the sensor 106 and extends back towards the proximal end 1122 through the cutter lumen 1154.

Figure 11O is a view showing the floss 1102 entering the opening 1124 of the window tube 1108 in accordance with embodiments herein. The floss 1102 extends through the bumper 1106 and into the hole 1104 in the sensor 106. The bumper 1106 may include relief features (e.g., notches, holes) to assist the exit of the floss 1102 and interface with the sensor 106.

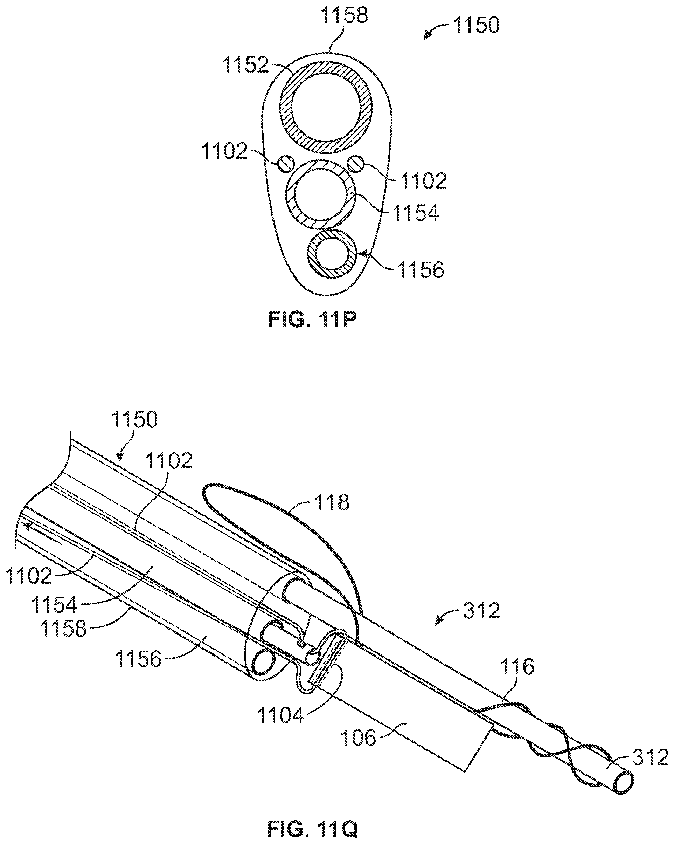

Figure 11P is a cut-away end view showing the tri-lumen catheter 1150 in accordance with embodiments herein. The GWL lumen 1152, the cutter lumen 1154, and the pressure lumen 1156 are shown within the heat shrink 1158 or other known bio-compatible material that holds the lumens together, keeps the lumens in the desired alignment with respect to each other, and/or supplies a smooth outer surface of the catheter. The floss 1102 extends in open area inside the confines of the heat shrink 1158 and not within any of the lumens 1152, 1154, 1156.

Figure 11Q illustrates another view of a sensor delivery system that utilizes the tri-lumen catheter to deliver the sensor within the body in accordance with embodiments herein. Figure 11Q shows the outer edge of the heat shrink 1158, and the heat shrink 1158 is transparent to show the GWL lumen 1152, the cutter lumen 1154, and the pressure lumen 1156. The floss 1102 extends within the heat shrink 1158, and not within any of the lumens 1152, 1154,1156. The floss 1102 is routed through the opening 1124 of the window tube 1108 as shown in Figure 110, and can exit from under a bumper 1106 to extend through the hole 1104 in the sensor 106.

### Full Length Tether Floss Release

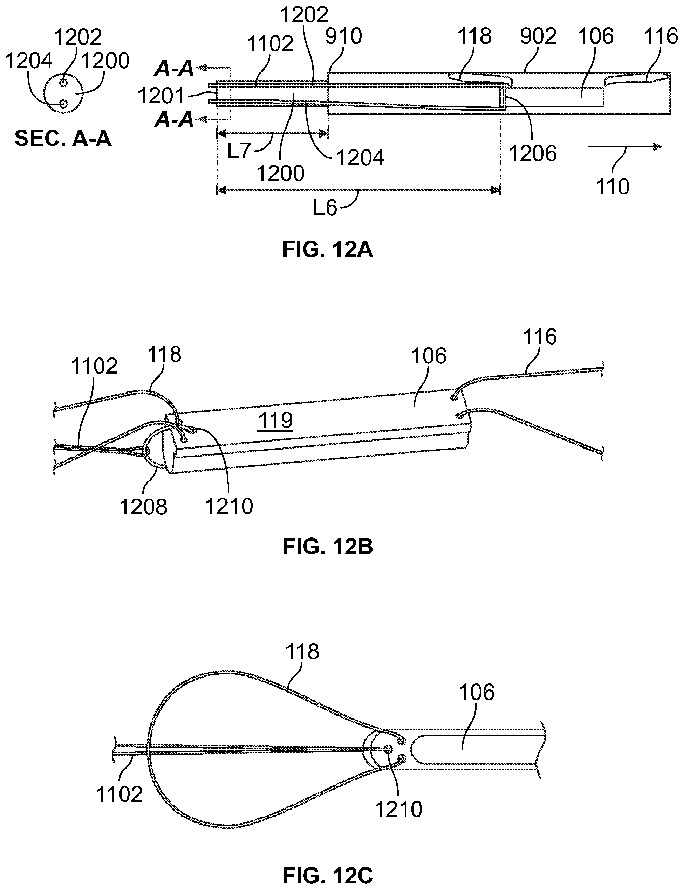

Figure 12A is a cross-sectional view of a push rod 1200 with the floss 1102 extending therethrough and removably coupling the sensor 106 in accordance with embodiments herein. The push rod 1200 can be a multi-lumen push rod having first and second lumens 1202, 1204 through which the full-length floss 1102 extends. For example, the floss 1102 runs from proximal of a proximal end 1201 of the push rod 1200, through one of the lumens 1202 and through a loop or hole 1206 in the sensor 106. The floss 1102 then extends back to the proximal end 1201 of the push rod 1200 through the other lumen 1204. It should be understood that the push rod 1200 can include an extra lumen to accommodate the GWL 312, and thus the distal loop 116 can be interleaved around the outside of the GWL 312 for increased stability.

The push rod 1200 extends a length L7 beyond the proximal end 910 of the delivery sheath 902 such that the push rod 1200 is longer than the delivery sheath 902 to ensure sensor exit when the push rod 1200 is pushed in the distal direction 110. When the sensor 106 is pushed out of the delivery sheath 902 at the desired deployment location, the practitioner pulls the floss 1102 a length L6 of the delivery system. In some embodiments, the practitioner may pull the floss 1102 approximately 60-120 cm, such as to pull the floss 1102 completely out of the hole 1206. In other embodiments, the practitioner may pull the floss 1102 completely out of the system, doubling the length of pulling (e.g., 120-240 cm).

In some cases, the floss 1102 can be a polymer suture (Polyester, Polyethylene, nylon, bioabsorbable, etc.) or Nitinol. Although a multi-lumen push rod 1200 is shown in Figure 12A, in other embodiments the push rod 1200 can have a single lumen. An advantage of the multi-lumen embodiment is that by holding each suture (e.g., half of the full-length floss 1102) in its own lumen 1202, 1204, the sutures will not twist which will control the pull forces as the floss 1102 is pulled to release the sensor 106.

Figure 12B illustrates the sensor 106 having a ring 1208 securely extending through a hole 1210 in the sensor 106 in accordance with embodiments herein. The floss 1102 is held by the ring 1208. The floss 1102 can extend through one or two lumens of the catheter as discussed. In some embodiments, the floss 1102 can be the release mechanism while the ring 1208 is the attachment feature that can extend through a hole in the body 119 of the sensor 106.

Figure 12C illustrates the sensor 106 wherein the floss 1102 extends through the hole 1210 in the sensor 106 in accordance with embodiments herein. Therefore, in some embodiments the hole 1210 is the attachment feature of the sensor 106. In some embodiments, utilizing the ring 1208 of Figure 12B or other feature to hold the floss 1102 may minimize drag when the floss 1102 is pulled compared to extending the floss 1102 through the hole 1210 in the sensor 106. Whether the ring 1208 is used or the floss extends through the hole 1210, the floss 1102 can be removed as discussed previously in Figure 12A.

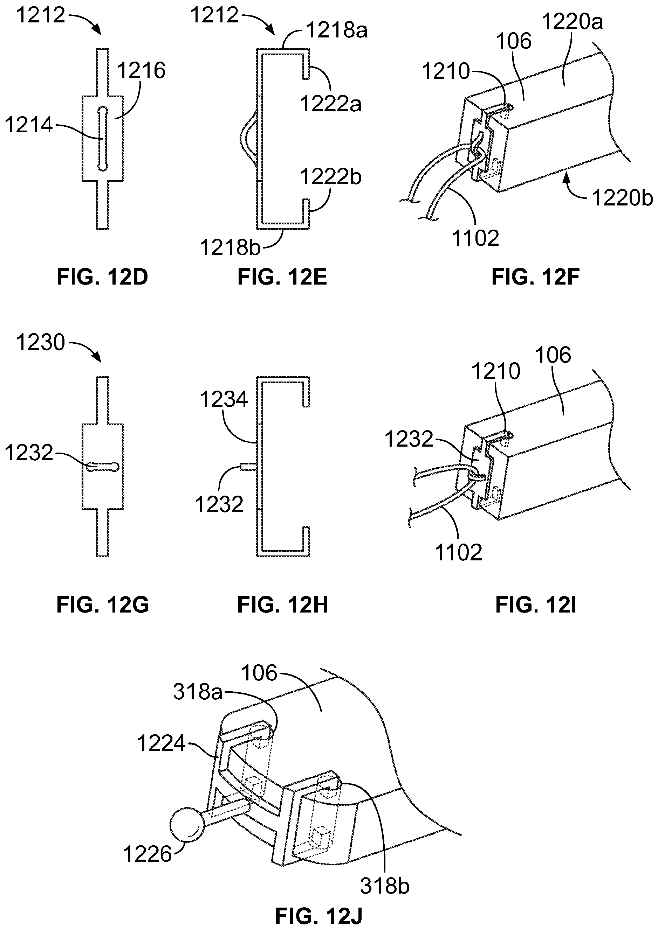

Alternative shapes of rings and/or other attachment features/fasteners can be used to removably couple with the floss 1102. Figures 12D-12F illustrate views of a floss loop 1212 formed in accordance with embodiments herein. A front view of the floss loop 1212 is shown in Figure 12D. In some embodiments, the floss loop 1212 can be a flat laser cut piece of material with a ring 1214 that extends from a body 1216. The ring 1214 can be formed integral with the body 1216, attached, or otherwise welded thereon, etc. The ring 1214 is configured to accept the floss 1102. In Figure 12E, the floss loop 1212 is shown in profile. Horizontal portions 1218a, 1218b are configured to extend over opposite sides 1220a, 1220b of the sensor 106. Vertical portions 1222a, 1222b are configured to extend from the horizontal portions 1218a, 1218b to enter opposite sides of the hole 1210, or into distinct holes on the opposite sides 1220a, 1220b of the sensor 106. Figure 12F illustrates the floss loop 1212 mounted on the sensor 106 with the floss 1102 extending through the ring 1214 in a horizontal direction.

Figures 12G-12I illustrate views of another floss loop 1230 formed in accordance with embodiments herein. A front view of the floss loop 1230 is shown in Figure 12G. The floss loop 1230 can be formed similar to the floss loop 1212 with the exception of the orientation of ring 1232. Figure 12H shows the side-profile of the floss loop 1230 with the ring 1232 extending outwardly at an approximately center location of body 1234, but the placement of the ring 1232 is not so limited. Figure 12I illustrates the ring 1232 mounted on the sensor 106 with the floss 1102 extending through the ring 1232 in a vertical direction. As shown in the example of attachment feature 1224 in Figure 12J, the floss loop can be formed to mount within the existing holes (e.g., attachment points 318 as shown in Figure 5A) in the sensor 106 that secure the anchor loops, instead of the two holes 1210 as shown in Figures 12F and 12I.

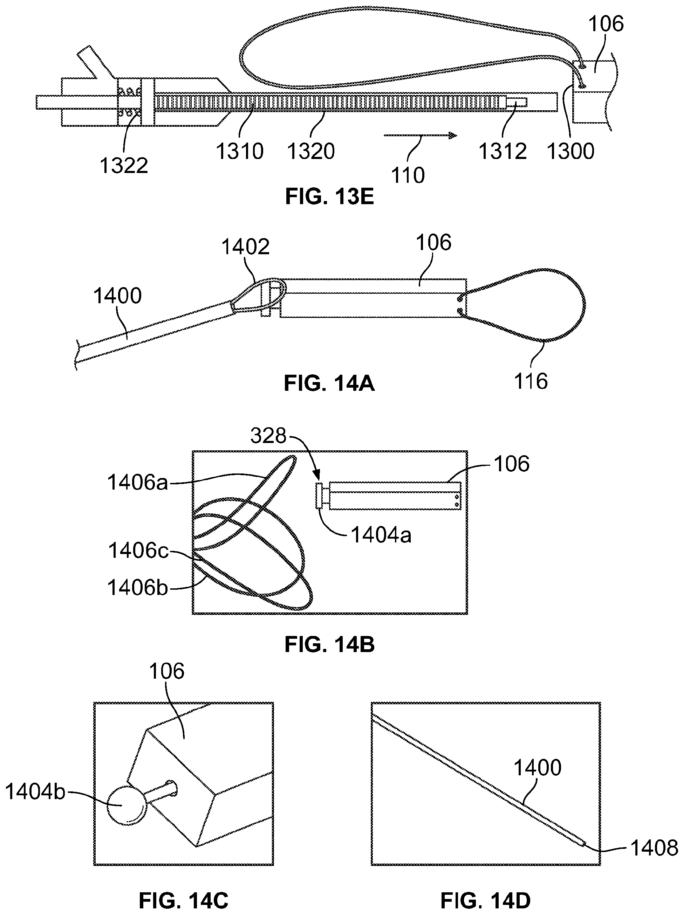

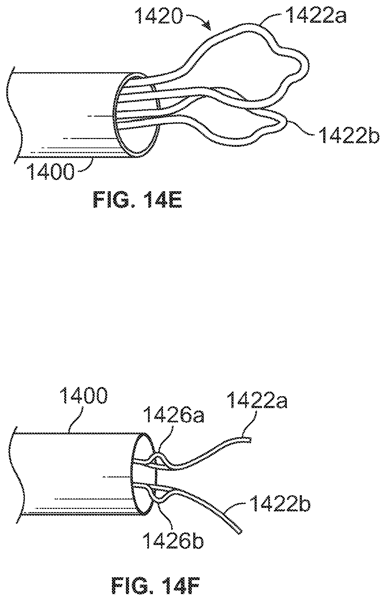

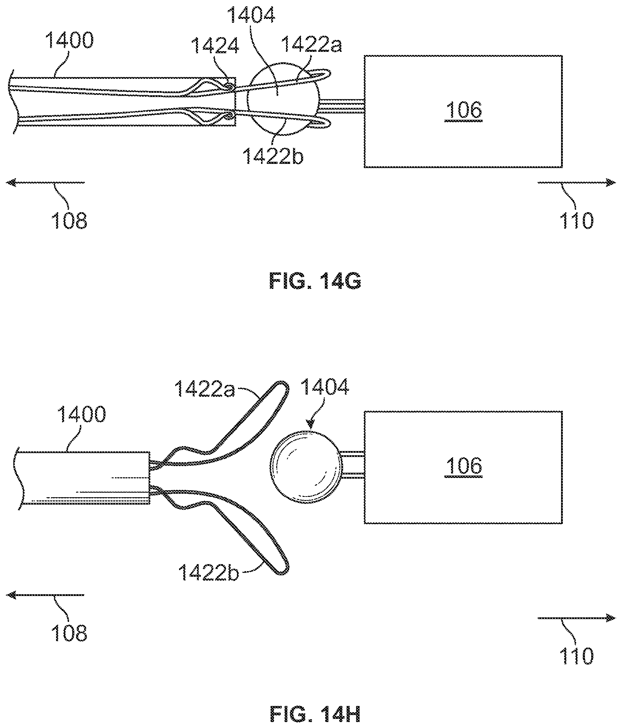

Figure 12J illustrates another view wherein an attachment feature 1224 can be mounted within the existing holes (e.g., attachment points 318 as shown in Figure 5A) in the sensor 106 that secure the anchor loops (not shown) in accordance with embodiments herein. Therefore, no further hole (e.g., hole 1210) within the sensor 106 would be needed, providing a manufacturing and assembly advantage as additional hole(s) would not need to be formed/drilled, space does not need to be devoted to the additional hole, etc. The attachment feature 1224 has a protrusion 1226 that can be used together with a snare or paddle feature as discussed herein, such as but not limited to in Figures 14.

### Shrouded Threaded Fastener Release

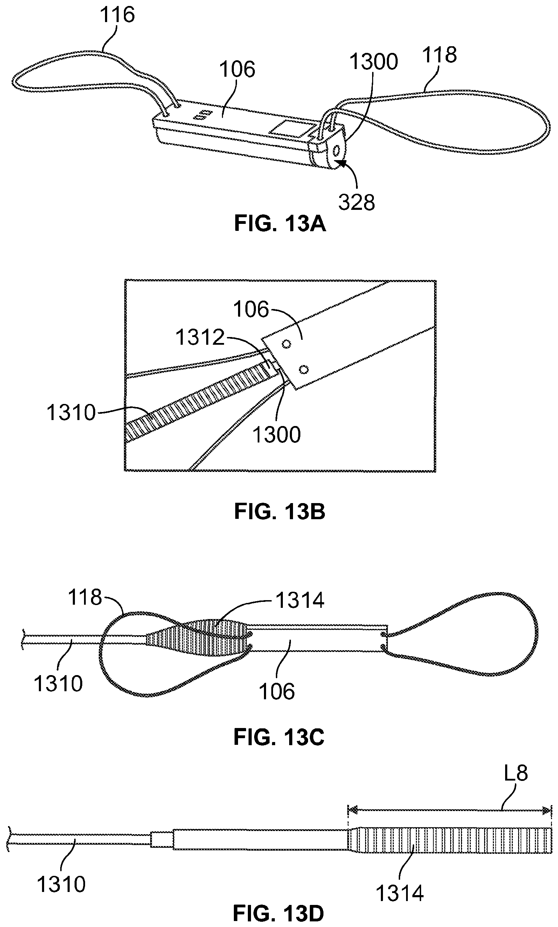

Figures 13A-13E illustrate additional embodiments wherein a threaded end of a threaded push rod or threaded fastener 323 of a torque cable 321 is protected by a sleeve or shroud after the sensor 106 is delivered in accordance with embodiments herein. Figure 13A illustrates the sensor 106, which in some embodiments is a PAP sensor as discussed previously. The sensor 106 has a threaded hole 1300 in the proximal end 328 of the sensor 106. In some embodiments, the threaded hole 1300 can be formed within the body of the sensor 106, while in other embodiments, the threaded hole 1300 may be formed in an additional component or attachment feature that is securely attached to the sensor 106.

Figure 13B shows the sensor 106 mated with a threaded end 1312 of a threaded push rod 1310. The threaded end 1312 is located at the distal end of the threaded push rod 1310. A portion of uncovered threads of the threaded end 1312 is shown, while a portion of the threads are threaded into the threaded hole 1300 in the sensor 106.

Figure 13C illustrates the threaded end 1312 of the push rod 1310 covered by a shroud 1314 in accordance with embodiments herein. During assembly, a sleeve or shroud 1314, such as a Nitinol braided sleeve, can be in a compressed/shortened state and covers the distal end of the threaded push rod 1310, and can extend a short distance proximally to secure the shroud 1314 to the threaded push rod 1310. In some embodiments, the shroud 1314 extends around the proximal end 328 of the sensor 106. When the threaded end 1312 is removed from the threaded hole 1300, such as by twisting or rotating the threaded push rod 1310, the length L8 of the shroud 1314 is long enough to cover the threaded end 1312 when the sensor 106 is released.

Figure 13D illustrates the threaded push rod 1310 and shroud 1314 after the threaded end 1312 of the push rod 1310 is released from the sensor 106 in accordance with embodiments herein. The shroud 1314 (e.g., uncompressed or free state) has a length L8 configured to cover at least the threaded end 1312 of the push rod 1310 when not mated with the sensor 106. The shroud 1314 (e.g., Nitinol braided sleeve) springs back to cover the threaded end 1312. The advantage of covering the threaded end 1312 is that the shroud 1314 acts as an additional barrier against accidental puncture of vessels. It should be understood that the shroud 1314 can be longer or shorter that shown with respect to the length of the push rod 1310. Further, an advantage of using a shape memory material to form the shroud 1314 (e.g., Nitinol) is that the shroud 1314 can be expanded or stretched to encircle the outer portion of the proximal end 328 of the sensor 106, and then return to its original shape to securely cover the threaded end 1312 while remaining securely attached to the threaded push rod 1310.

Figure 13E illustrates another embodiment wherein the threaded push rod 1310 is substantially covered by a full-length sleeve 1320 in accordance with embodiments herein. The sleeve 1320 can be a polymer tube that is spring loaded, such as with spring 1322, that is located near the proximal end of the threaded push rod 1310. In some embodiments the spring 1322 can be located closer to the distal end of the threaded push rod 1310. The spring 1322 is compressed when the sensor 106 is loaded by screwing the threaded end 1312 into the threaded hole 1300. When the sensor 106 is released by unscrewing the threaded end 1312 of the sensor 106 from the threaded hole 1300, the spring 1322 pushes in the distal direction 110 and pushes the sleeve 1320 over the threaded end 1312 of the push rod 1310. Accordingly, if the push rod 1310 is accidentally readvanced after deploying the sensor 106, such as in the pulmonary artery, the threaded end 1312 is covered and will not puncture or otherwise damage the vessel.

### Ball and Snare Release

Figures 14A-14D illustrate embodiments wherein a snare contained within a pushrod 1400 interconnects with a protruding attachment feature on the sensor 106 in accordance with embodiments herein. In some embodiments the pushrod 1400 can be a hollow tube. The pushrod 1400 can be included within any of the single and/or multi-lumen catheters and or pressure sheath as discussed herein. Figures 14A-B illustrate utilizing a tri-snare 1402. However, it should be understood that the snare can have one loop (e.g., discussed in Figure 14C) or two loops (not shown). The protrusion 1404a in Figure 14B is mounted on and/or protrudes proximally from the proximal end 328 of the sensor 106 and can, for example, have radiused triangle points (not shown). Each loop 1406a, 1406b, 1406c of the tri-snare 1402 catches on one triangle point of the protrusion 1404a. In other embodiments a grooved adapter (not shown) having one or more grooves thereon can be mounted to the proximal end 328 of the sensor 106, which is then snared by one or more loops. The protrusion 1404b in Figure 14C can be a ball, round-head pin, or other geometric shape that can be captured by a single loop snare (not shown). In some cases, using three loops, such as the tri-snare 1402, can provide the advantage of keeping the sensor 106 in a more centered position. To release the sensor 106, the tri-snare 1402 can be extended in the distal direction 110, causing the loops 1406 to expand outwardly away from the protrusion. Figure 14D is a view of the pushrod 1400 wherein the snare is retracted into a distal end 1408 of the pushrod 1400 after the sensor 106 has been released.

Figure 14E shows a snare 1420 within a pushrod 1400 that includes multiple detent paddles 1422a, 1422b in accordance with embodiments herein. The detect paddles 1422 are configured to grasp a protrusion 1404 on the proximal end of the sensor 106, such as the protrusion 1404b as shown in Figure 14C, but is not limited to a spherical protrusion. As shown, in Figure 14E, detent paddle 1422a is substantially opposite of detent paddle 1422b, but is not so limited. In some embodiments there can be three detent paddles 1422 arranged triangularly with respect to each other, while in other embodiments there can be four detent paddles 1422 wherein each detent paddle 1422 has another detent paddle 1422 that is positioned substantially opposite.

Although the detent paddles 1422 are shown as having a circular or round opening with which to grasp the protrusion 1404, the opening can be shaped differently to accommodate differently shaped protrusions 1404. Also, the detent paddles 1422 can be substantially flat, have distal tips that curve outward or inward, and the like.

Figure 14F shows a side view of the detent paddles 1422 in accordance with embodiments herein. The pushrod 1400 has been retracted and thus the detent paddles 1422 are in their fully extended or open state, or the position furthest from each other. In some embodiments, the detent paddles 1422 may be formed of a shape memory material that retains the open position, or position the detent paddles 1422 will be in when the protrusion 1404 is released, unless under pressure from the pushrod 1400. One or more of the detent paddles 1422 can include a protrusion or bend 1426, such that when the pushrod 1400 is extended in the distal direction 110, contact of the bend(s) 1426 with the inner surface of the pushrod 1400 cause the detent paddles 1422 to move inward or toward each other.

Figure 14G shows the detent paddles 1422 engaged with the protrusion 1404 in accordance with embodiments herein. The detent paddles 1422 can be brought closer to each other to fully engage the protrusion 1404 by moving the detent paddles 1422 relative to a distal end 1424 of the pushrod 1400. In other words, the pushrod 1400 squeezes the detent paddles 1422 closer to each other. The detent paddles 1422 may be locked into position, such as by locking the wire or other actuator that is connected to the detent paddles 1422 within the pushrod 1400 to prevent unintended release of the sensor 106.

Figure 14H shows the detent paddles 1422 disengaged from the protrusion 1404 of the sensor 106 in accordance with embodiments herein. Once the sensor 106 is positioned within the vessel, the pushrod 1400 can be retracted in the proximal direction 108 relative to the detent paddles 1422. In some embodiments the detent paddles 1422 are pushed in the distal direction 110. When the pushrod 1400 is retracted, the detent paddles 1422 spring away from each other, releasing the protrusion 1404.

### Expanding Detents Release

Figures 15A-15G illustrate embodiments wherein a locking feature at a distal end of push rod 1500 can be removably coupled to a nut 1506 having a recess or pocket 1507 attached to and/or integral with the proximal end 328 of the sensor 106 in accordance with embodiments herein. Referring to Figure 15A, the locking feature on the push rod 1500 can include detents 1502 such as fingers (e.g., Nitinol fingers) and a nut or ball 1504 (e.g., rubber ball). The detents 1502 can be formed integral with a sheath that is part of the push rod 1500, formed at a proximal end of the push rod 1500, and the like. The ball 1504 can be moved with respect to the detents 1502 by, for example, pushing the push rod 1500 (or another additional rod) in the distal direction 110 and pulling the push rod 1500 (or the additional rod) the proximal direction 108. When the ball 1504 is pulled back into the detents 1502, the ball 1504 is stuck until the ball 1504 is advanced out of the detents 1502.

The nut 1506 can have an opening 1508 that accepts diameters associated with the detents 1502 and the ball 1504 when the detents 1502 and the ball 1504 are not engaged, as shown in view 1510. Therefore, the ball 1504 and detents 1502 can be separately advanced into the pocket 1507. The opening 1508 is not wide enough to allow the diameter of the expanded detents 1502 holding the ball 1504 to pass through, as shown in view 1512, thus capturing the locking feature.

Figure 15B illustrates an end view 1514, a cross-sectional side view 1516, and a cross-sectional top view 1518 of the nut 1506 and pocket 1507 mated or integral with the sensor 106. The cross-sectional side view 1516 and the cross-sectional top view 1518 indicate several different ways the nut 1506 and pocket 1507 can integrate with the sensor 106.

Figures 15C, 15D are similar to Figures 15A, 15B. In Figure 15C, the detents 1502 are positioned within the opening 1508 and the ball 1504 is pulled in the proximal direction108 into the detents 1502. The detents expand outwardly (e.g., having a first larger diameter) to create an interference fit that secures the sensor 106 to the distal end of the push rod 1500. When the ball 1504 is pushed in the distal direction 110, the ball 1504 moves out of the detents 1502 and the detents 1502 return to their unexpanded shape (e.g., having a second smaller diameter). By disengaging the detents 1502 and the ball 1504, there is enough clearance to pull the push rod 1500 in the proximal direction 108 to remove the detents 1502 and ball 1504 from the nut 1506 and pocket 1507 and release the sensor 106 (Figure 15D).

Figures 15E-15G illustrate alternative embodiments wherein the locking feature (e.g., release mechanism) can include different shapes to create the interference fit with the nut 1506 and pocket 1507 (e.g., attachment feature) in accordance with embodiments herein. Figure 15E has formed knuckles 1528 and 1530 mounted on separate wires 1532, 1534 (or ribbons) that can slide to reduce the profile. Figures 15F and 15G show balls 1520 and 1522 mounted on separate wires 1524, 1526. The wires 1524, 1526 can be pulled in the proximal direction 108 or pushed in the distal direction 110 to move the balls 1520, 1522 relative to each other. When the balls 1520, 1522 are aligned as shown in Figure 15F, the locking feature forms an interference fit within the nut 1506 and/or within the entry of the nut 1506. When the balls 1520, 1522 are not aligned as shown in Figure 15G, the locking feature can be removed from the nut 1506, releasing the sensor 106 from the push rod 1500. Figure 15E can generally be referred to as sliding fingers and operates in the same manner as described for Figures 15F and 15G.

### Jaws, Paddles, and/or Slides Release

The embodiments of 16A-16H utilize a nut (attachment feature) bonded onto the sensor 106 that has one or more recess/hole specifically designed to interface with an attachment mechanism (e.g., jaw, paddle, or slide) of a push rod to form a locking feature in accordance with embodiments herein. In some cases, the one or more recess can be provided in the sensor 106 itself. In some embodiments the jaws and paddles are made from shape memory material(s) such as Nitinol so that when the sensor 106 is advanced out of the sheath(s) or the sheath compressing the paddles/jaws is retracted, the arms of the paddles/jaws open up and release the sensor 106. In some cases, a flat Nitinol ribbon can be cut into a desired shape, such as a paddle, clamp arm, etc., extending at the end of a wire that can also be Nitinol. Some embodiments may not require self-expanding materials, while some may require a second action, such as pulling action in the proximal direction 108 to release the sensor 106.

Figures 16A and 16B illustrate a push rod 1601 with a sleeve 1600 or sheath around wires 1602a, 1602b that are attached to, integral with, and/or terminate with clamp arms 1604a, 1604b in accordance with embodiments herein. When the sleeve 1600 is forward (e.g., pushed in the distal direction 110), distal portions of the clamp arms 1604 hold securely to the sensor 106. When the sleeve 1600 is pulled back (e.g., pulled in the proximal direction 108), the attachment feature of the sensor 106 advances distally out of the delivery sleeve 1600, and distal portions of the clamp arms 1604 flex outwardly to release the sensor 106. In other words, when the sensor 106 is advanced out of the sheath(s), the clamp arms 1604 open.

Figure 16C illustrates a nut 1612 with recesses 1610a, 1610b formed on opposite sides configured to receive jaws or paddles in accordance with embodiments herein. The nut 1612 is attached to the proximal end of the sensor 106. The recess 1610 can have a ramp feature 1614 to engage the attachment mechanism at an angle as the clamp arms 1604 are compressed by the sleeve 1600.

Figure 16D illustrates flat paddles 1620a, 1620b configured to interface with the recesses 1610a, 1610b (e.g., pockets, etc.) when the sleeve 1600 is pushed forward (e.g., distal direction 110) to lock the paddles 1620 and recesses 1610 together. The paddles 1620a, 1620b can have a pre-set bend to ensure locking and/or can be configured to slightly bend under pressure. Figure 16E illustrates the flat paddles 1620a, 1620b disengaged from the recesses 1610a, 1610b in the nut 1612. Figure 16F illustrates an embodiment having jaws 1640a, 1640b that are operable in a similar manner. In some embodiments, the recesses 1610a, 1610b can include an additional hole 1642 extending into the nut 1612 configured to engage a tip portion 1644 (e.g., protrusion, fang, etc.) of the jaws 1640.

Figure 16G illustrates a nut 1652 that has windows or openings 1654a, 1654b on opposite sides for accepting slides 1650a, 1650b in accordance with embodiments herein. The nut 1652 is attached to and/or integral with the proximal end 328 of the sensor 106. The slides 1650 are attached to a push rod 1656. The slides 1650a, 1650b extend through the openings 1654a, 1654b and can be attached to a sleeve or sheath (not shown) that extends proximally over at least a portion of the push rod 1656. When the sheath is pulled in the proximal direction 108, the slides 1650 are pulled from the openings 1654 while the push rod 1656 is held in place until the sensor 106 is deployed, as shown in Figure 16H.

Figures 16I-16L illustrate further embodiments for securing the sensor 106 to the push rod 1601 until deployment in accordance with embodiments herein. Figure 16I illustrates tension hooks 1660a, 1660b that can be used with a spring (not shown) or formed of a shape-retaining material such as Nitinol, etc. The tension hooks 1660 can interface with holes 1662, protrusions and/or the like in an outer coating of the sensor 106. A sleeve 1600 can be pulled in the proximal direction 108, causing the tension hooks 1660 to expand outwardly, lift from and release the sensor 106. In other embodiments, release from the sensor 106 may be accomplished using a spring located at the proximal end of the push rod 1601, as discussed above in Figure 13E.

Figure 16J illustrates a flange 1672 that can interface with arms 1670a, 1670b that are held within or by the push rod 1601. In one embodiment, the sleeve 1600 can be pulled in the proximal direction 108 to allow the arms 1670a, 1670b to deflect outwardly to be released from a distal side of the flange 1672.

Figure 16K illustrates paddles 1680a, 1680b that can be configured to capture the proximal loop 118 when the push rod 1601 and sensor 106 are mated together. Again, in some embodiments the sleeve 1600 can be pulled in the proximal direction 108, allowing the paddles 1680a, 1680b to open enough to release the proximal loop 118. An advantage of this embodiment is that no change is needed with respect to the sensor 106; in other words, the attachment of the sensor 106 is the proximal loop 118.

Figure 16L illustrates grasping arms 1690a, 1690b that are configured to be held within the push rod 1601 and removably couple with a loop 1692 attached to the sensor 106 (e.g., the ring 1214, 1232 as shown in Figure 12F, 12I). Again, the grasping arms 1690a, 1690b can be held close together with the sleeve 1600 to mate the push rod 1601 and the sensor 106, and separated by pulling the sleeve 1600 in the proximal direction 108.

### Pinch Clamp Release

Figures 17A-17B illustrate a pinch clamp 1700 configured to removably couple the sensor 106 to push rod 1702 in accordance with embodiments herein. The pinch clamp 1700 can hold the sensor 106 concentrically in the delivery sheath. In the example of Figure 17, a special pocket (e.g., nut) or sensor feature (e.g., holes, etc.) may not be needed. Referring to the top-down depiction in Figure 17A, the pinch clamp 1700 has first and second clamps 1704a, 1704b that are fitted to the body of the sensor 106 at both proximal and distal ends 328, 330. Alternatively, the clamps 1704 can be fitted along either side of the sensor body 119, as shown in Figure 17B. A common wire 1706 extends between the clamps 1704a, 1704b. The wire 1706 is tensioned to hold the clamps 1704a, 1704b around the body 119 of the sensor 106. Once the sensor 106 has been pushed out of the sheath (not shown) by the push rod 1702, a wire in the push rod 1702 can be pushed in the distal direction 110 to decrease the tension on the wire 1706 to release the sensor 106 (e.g., clamps 1704 open enough to release the sensor 106). The clamps 1704 can then be retracted into the delivery sheath and/or push rod 1702. In some embodiments, each of the clamps 1704 can be attached to a separate tether in the push rod 1702 or in an associated catheter within the delivery system.

### Bumper Release

All of the sheathed delivery release mechanisms described herein can be combined with some form of bumper on the push rod/catheter so that if a case is ever aborted, the bumper CAN help make the sensor 106 coaxial and easier to retrieve into the delivery sheath or introducer. In some embodiments, the bumper can have a flared shape that interfaces with the sensor 106.

Figure 18A illustrates a cross-sectional view of a bumper 1800 on a distal end 1804 of a push rod 1802 in accordance with embodiments herein. Distal end 1806 of the bumper 1800 interfaces with the sensor 106. In some embodiments, an attachment mechanism (e.g., any of the release mechanisms described herein) can protrude through and/or around the bumper 1800 to releasably couple the push rod 1802 with an attachment feature of the sensor 106.

To retrieve the sensor 106, the bumper 1800 can be positioned against the proximal side of the sensor 106. The shape of the distal end 1806 of the bumper 1800, which in some cases can be flared and/or shaped to match a particular sensor geometry and/or size, can assist with holding the sensor 106 coaxial with the delivery sheath or introducer. Known retrieval methods can be used, which is some cases can be a reversal of the release mechanism, or by inserting a different tool.

Figure 18B illustrates a cross-sectional view of a bumper 1810 that can be used together with a snare 1812 to retrieve the sensor 106. For example, the bumper 1810 can be used together with embodiments as discussed above in Figure 14A-14D. As with the bumper 1800 of Figure 18A, the bumper 1810 can be positioned around the push rod 1802 and can be actuated with a wire, sheath interconnection, and the like.

Figure 18C illustrates an alternative catheter 1850 that is shaped to guide removal of the sensor 106 through the delivery sheath in accordance with embodiments herein. In some embodiments, the shape of the catheter 1850 can be configured to include a bumper with a protrusion 1852 on the distal end configured to interface with the distal loop 116 for retrieval. The catheter can have a narrow portion 1854 that extends the length of the sensor 106 with protrusions 1856a, 1856b extending outward to rest against either end of the sensor 106. In some embodiments the catheter 1850 can be extruded with the desired shape and/or be mated with a separate molded component. Figure 18D illustrates a catheter 1840 with a cut out 1872 in accordance with embodiments herein. The sensor 106 can be recessed into one side of the catheter 1840 for retrieval.

### System Utilizing Implantable Sensor

Figure 19 illustrates a system 1901 that includes an implantable medical device (IMD) 1900, an implantable sensor 1950, and an external device 1904 implemented in accordance with embodiments herein. The IMD 1900 and the implantable sensor 1950 (e.g., the sensor 106 described above) are implanted within the body of a patient. The external device 1904 is outside of the patient body. The external device 1904 may be a programmer, an external defibrillator, a workstation, a portable computer (e.g., laptop or tablet computer), a personal digital assistant, a cell phone (e.g., smartphone), a bedside monitor, a wand, a pillow or other product that the patient can lay against and/or rest against their body, and the like. The IMD 1900 may represent a cardiac monitoring device, a pacemaker, a cardioverter, a cardiac rhythm management device, a defibrillator, a neurostimulator, a leadless monitoring device, a leadless pacemaker, and the like, implemented in accordance with one embodiment of the present invention. The IMD 1900 may be a dual-chamber stimulation device capable of treating both fast and slow arrhythmias with stimulation therapy, including cardioversion, defibrillation, anti-tachycardia pacing and pacing stimulation, as well as capable of detecting heart failure, evaluating its severity, tracking the progression thereof, and controlling the delivery of therapy and warnings in response thereto. The IMD 1900, implantable sensor 1950, and external device 1904 can be found, for example, in U.S. Patent Application serial number 17/820,654 entitled "System and Method for Intra-Body Communication of Sensed Physiologic Data", filed on August 18, 2022.

The IMD 1900 includes a housing 1906 that is joined to a header assembly 1908 that holds receptacle connectors connected to a right ventricular lead 1930 and an atrial lead 1920, respectively. The atrial lead 1920 includes a tip electrode 1922 and a ring electrode 1923. The right ventricular lead 1930 includes an RV tip electrode 1932, an RV ring electrode 1934, an RV coil electrode 136, and an SVC coil electrode 1938. The leads 1920 and 1930 detect intracardiac electrogram (IEGM) signals that are processed and analyzed, and also deliver therapies.

The IMD 1900 may be implemented as a full-function biventricular pacemaker, equipped with both atrial and ventricular sensing and pacing circuitry for four chamber sensing and stimulation therapy (including both pacing and shock treatment). Optionally, the IMD 1900 may further include a coronary sinus lead with left ventricular electrodes. The IMD 1900 may provide full-function cardiac resynchronization therapy. Alternatively, the IMD 1900 may be implemented with a reduced set of functions and components. For instance, the IMD may be implemented without ventricular sensing and pacing.

The implantable sensor 1950 is configured to be implanted at a location remote from the electrodes of the leads 1920 and 1930. The implantable sensor 1950 may be implanted in a blood vessel, such as an artery or vein. In an embodiment, the sensor 1950 is implanted within the pulmonary artery (PA). The sensor 1950 may be anchored to the vessel wall of a blood vessel using one or more expandable loop wires. The diameter of each loop should be larger than the diameter of target blood vessel in order to provide adequate anchoring force. Optionally, instead of the loop wire, the sensor 1950 may be attached to the end of a self-expandable stent and deployed into the blood vessel through a minimally invasive method.

Alternatively, the implantable sensor 1950 may be secured to tissue outside of blood vessels. The sensor 1950 may be secured in place by using a fixation screw (e.g., helix) attached to the housing. The screw may anchor the sensor 1950 to patient heart tissue, such as cardiac tissue of the left or right ventricle. The sensor 1950 is configured to sense a physiologic parameter of interest (PPOI) and to generate signals indicative of the PPOI. In a non-limiting example, when the sensor 1950 is disposed within the PA, the sensor 1950 may sense, as the PPOI, blood pressure.

In some embodiments, the sensor 1950 can be powered by, and communicate with, the external device 1904. In other embodiments, the sensor 1950 can communicate with the IMD 1900. The sensor 1950 can communicate information about the physiologic parameter, for example.

### Closing

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random access memory (RAM), a dynamic random access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the Figures, which illustrate example methods, devices and program products according to various example embodiments. These program instructions may be provided to a processor of a general purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only, and are thus not intended to limit in any way the definition and/or meaning of the term "controller." The units/modules/applications herein may execute a set of instructions that are stored in one or more storage elements, in order to process data. The storage elements may also store data or other information as desired or needed. The storage elements may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. An intracorporeal sensor delivery system (100, 300) comprising:
a delivery catheter (102, 306) comprising a lumen (308), the delivery catheter (102, 306) having a proximal end (332) and a distal end (334);
a guide wire lumen (GWL) (112, 312) configured to receive a guide wire (114, 322), the GWL (112, 312) extending within the lumen (308) of the delivery catheter (102, 306) and protruding beyond the distal end (334) of the delivery catheter (102, 306);
an intracorporeal sensor (106) comprising a proximal end (328) and a distal end (330), wherein the proximal end (328) of the intracorporeal sensor (106) is positioned distal to the distal end (334) of the delivery catheter (102, 306);
a proximal coupling feature (118, 316) coupled to the proximal end (328) of the intracorporeal sensor (106), the proximal coupling feature (316) configured to removably couple the intracorporeal sensor (106) to the delivery catheter (102, 306); and
a distal coupling feature (116, 324) coupled to the distal end (330) of the intracorporeal sensor (106), **characterized in that** the distal coupling feature (324) further removably coupled around an outer surface of the GWL (112, 312) at a position that is distally located with respect to the distal end (330) of the intracorporeal sensor (106).

2. The system of claim 1, wherein the distal coupling feature (116, 324) comprises a loop (116, 324) that is interlaced around the outer surface of the GWL (112, 312).

3. The system of claim 2, wherein the loop (116, 324) comprises:
a first loop portion (406) attached to the intracorporeal sensor (106) at a first attachment point (326a); and
a second loop portion (408) attached to the intracorporeal sensor (106) at a second attachment point (326b), wherein the first and second loop portions (406, 408) of the distal coupling feature (116, 324) are interlaced to form a first set of cross points (402a, 402b, 402c) along a first side of the GWL (112, 312) and a second set of cross points (404a, 404b, 404c) along a second side of the GWL (112, 312), wherein the first loop portion (406) is outside of the second loop portion (408) at the first set of cross points (402a, 402b, 402c) and the second loop portion (408) is outside of the first loop portion (406) at the second set of cross points (404a, 404b, 404c).

4. The system of claim 1, wherein the distal coupling feature (116, 324) comprises a loop (116, 324) that is twisted or wrapped around the outer surface of the GWL (112, 312).

5. The system of claim 1, wherein the proximal coupling feature (118, 316) comprises a loop (118, 316) that is twisted at least once around itself.

6. The system of claim 1, wherein the GWL (112, 312) comprises a length, wherein a first portion (386, 388) of the length has a first stiffness, and a second portion (390) of the length has a second stiffness that is different from the first stiffness.

7. The system of claim 1, wherein the distal coupling feature (116, 324) comprises a loop (116, 324) that is wrapped around the outer surface of the GWL (112, 312) to allow, in response to the GWL (112, 312) being decoupled from the distal_coupling feature (116, 324), the loop (116, 324) to open laterally with no axial twisting, the opened loop (116, 324) configured to engage walls of a vessel to provide rotational stability of the intracorporeal sensor (106).

8. The system of claim 1, further comprising a pressure sheath (120, 600) configured to removably cover a portion of the delivery catheter (102, 306) and a portion of the proximal coupling feature (118, 316).

9. The system of claim 1, wherein the intracorporeal sensor (106) is a pressure sensor.

10. The system of claim 1, wherein the delivery catheter (306) further comprises a second lumen (310) extending parallel with respect to the lumen (308).

11. The system of claim 10, further comprising a pressure lumen (313) positioned in parallel and held together with the second lumen (310) and the GWL (312), optionally wherein the second lumen (310) is configured to facilitate pressure readings.

12. The system of claim 10, wherein the intracorporeal sensor delivery system (300) further comprising:
a torque cable (321) extending through the second lumen (310), the torque cable (321) comprising a proximal coupling feature (323) configured to removably couple to the proximal end (328) of the intracorporeal sensor (106); or
a push rod (904) extending through the second lumen (310), the push rod (904) further comprising a cutting element or torque cable.

13. The system of claim 1, wherein the delivery catheter (102, 306) further comprises a skive (122, 314) into a lumen (308) positioned proximal to the distal end (334) of the delivery catheter (102, 306), the skive (122, 314) configured to receive the proximal coupling feature (118, 316), the proximal coupling feature (118, 316) comprising a loop (118, 316) extending through the skive (122, 314) and between the GWL (112, 312) and the delivery catheter (102, 306).

14. The system of claim 1, wherein the delivery catheter (102, 306) further comprises a skive (122, 314) positioned proximal to the distal end (334) of the delivery catheter (102, 306), the skive (122, 314) configured to receive the proximal coupling feature (118, 316), the proximal coupling feature (118, 316) comprising a loop (118, 316) interlaced around the outer surface of the GWL (112, 312).

15. The system of claim 1, wherein the proximal coupling feature (118, 316) is one of a floss, a threaded fastener, a snare, paddles, or detents.

## Patentansprüche

1. Freisetzungssystem (100, 300) für intrakorporale Sensoren, umfassend:
einen Freisetzungskatheter (102, 306), umfassend ein Lumen (308), wobei der Freisetzungskatheter (102, 306) ein proximales Ende (332) und ein distales Ende (334) aufweist;
ein Führungsdrahtlumen (GWL) (112, 312), das dazu konfiguriert ist, einen Führungsdraht (114, 322) aufzunehmen, wobei sich das GWL (112, 312) innerhalb des Lumens (308) des Freisetzungskatheters (102, 306) erstreckt und über das distale Ende (334) des Freisetzungskatheters (102, 306) vorspringt;
einen intrakorporalen Sensor (106), umfassend ein proximales Ende (328) und ein distales Ende (330), wobei das proximale Ende (328) des intrakorporalen Sensors (106) distal zu dem distalen Ende (334) des Freisetzungskatheters (102, 306) positioniert ist;
ein proximales Kopplungsmerkmal (118, 316), das an das proximale Ende (328) des intrakorporalen Sensors (106) gekoppelt ist, wobei das proximale Kopplungsmerkmal (316) dazu konfiguriert ist, den intrakorporalen Sensor (106) entfernbar an den Freisetzungskatheter (102, 306) zu koppeln; und
ein distales Kopplungsmerkmal (116, 324), das an das distale Ende (330) des intrakorporalen Sensors (106) gekoppelt ist, **dadurch gekennzeichnet, dass** das distale Kopplungsmerkmal (324) ferner entfernbar um eine Außenfläche des GWL (112, 312) an einer Position gekoppelt ist, die sich in Bezug auf das distale Ende (330) des intrakorporalen Sensors (106) distal befindet.

2. System nach Anspruch 1, wobei das distale Kopplungsmerkmal (116, 324) eine Schleife (116, 324) umfasst, die um die Außenfläche des GWL (112, 312) zwischenverbunden ist.

3. System nach Anspruch 2, wobei die Schleife (116, 324) Folgendes umfasst:
einen ersten Schleifenabschnitt (406), der an einem ersten Befestigungspunkt (326a) an dem intrakorporalen Sensor (106) befestigt ist; und
einen zweiten Schleifenabschnitt (408), der an einem zweiten Befestigungspunkt (326b) an dem intrakorporalen Sensor (106) befestigt ist, wobei der erste und der zweite Schleifenabschnitt (406, 408) des distalen Kopplungsmerkmals (116, 324) zwischenverbunden sind, um einen ersten Satz von Kreuzpunkten (402a, 402b, 402c) entlang einer ersten Seite des GWL (112, 312) und einen zweiten Satz von Kreuzpunkten (404a, 404b, 404c) entlang einer zweiten Seite des GWL (112, 312) zu bilden, wobei sich der erste Schleifenabschnitt (406) an dem ersten Satz von Kreuzpunkten (402a, 402b, 402c) außerhalb des zweiten Schleifenabschnitts (408) befindet und sich der zweite Schleifenabschnitt (408) an dem zweiten Satz von Kreuzpunkten (404a, 404b, 404c) außerhalb des ersten Schleifenabschnitts (406) befindet.

4. System nach Anspruch 1, wobei das distale Kopplungsmerkmal (116, 324) eine Schleife (116, 324) umfasst, die um die Außenfläche des GWL (112, 312) verdrillt oder gewickelt ist.

5. System nach Anspruch 1, wobei das proximale Kopplungsmerkmal (118, 316) eine Schleife (118, 316) umfasst, die mindestens einmal um sich selbst verdrillt ist.

6. System nach Anspruch 1, wobei das GWL (112, 312) eine Länge umfasst, wobei ein erster Abschnitt (386, 388) der Länge eine erste Steifigkeit aufweist und ein zweiter Abschnitt (390) der Länge eine zweite Steifigkeit aufweist, die sich von der ersten Steifigkeit unterscheidet.

7. System nach Anspruch 1, wobei das distale Kopplungsmerkmal (116, 324) eine Schleife (116, 324) umfasst, die um die Außenfläche des GWL (112, 312) gewickelt ist, um es als Reaktion darauf, dass das GWL (112, 312) von dem distalen Kopplungsmerkmal (116, 324) entkoppelt ist, der Schleife (116, 324) zu ermöglichen, sich seitlich ohne axiale Verdrehung zu öffnen, wobei die geöffnete Schleife (116, 324) dazu konfiguriert ist, Wände eines Gefäßes in Eingriff zu nehmen, um eine Drehstabilität des intrakorporalen Sensors (106) bereitzustellen.

8. System nach Anspruch 1, ferner umfassend eine Druckhülle (120, 600), die dazu konfiguriert ist, einen Abschnitt des Freisetzungskatheters (102, 306) und einen Abschnitt des proximalen Kopplungsmerkmals (118, 316) entfernbar abzudecken.

9. System nach Anspruch 1, wobei der intrakorporale Sensor (106) ein Drucksensor ist.

10. System nach Anspruch 1, wobei der Freisetzungskatheter (306) ferner ein zweites Lumen (310) umfasst, das sich parallel in Bezug auf das Lumen (308) erstreckt.

11. System nach Anspruch 10, ferner umfassend ein Drucklumen (313), das parallel zu dem zweiten Lumen (310) und dem GWL (312) positioniert und mit diesen zusammengehalten ist, gegebenenfalls wobei das zweite Lumen (310) dazu konfiguriert ist, Druckmesswerte zu erleichtern.

12. System nach Anspruch 10, wobei das Freisetzungssystem (300) für intrakorporale Sensoren ferner Folgendes umfasst:
ein Drehmomentkabel (321), das sich durch das zweite Lumen (310) hindurch erstreckt, wobei das Drehmomentkabel (321) ein proximales Kopplungsmerkmal (323) umfasst, das dazu konfiguriert ist, sich entfernbar an das proximale Ende (328) des intrakorporalen Sensors (106) zu koppeln; oder
eine Schubstange (904), die sich durch das zweite Lumen (310) hindurch erstreckt, wobei die Schubstange (904) ferner ein Schneideelement oder ein Drehmomentkabel umfasst.

13. System nach Anspruch 1, wobei der Freisetzungskatheter (102, 306) ferner einen Schärfer (122, 314) in ein Lumen (308) hinein umfasst, der proximal zu dem distalen Ende (334) des Freisetzungskatheters (102, 306) positioniert ist, wobei der Schärfer (122, 314) dazu konfiguriert ist, das proximale Kopplungsmerkmal (118, 316) aufzunehmen, wobei das proximale Kopplungsmerkmal (118, 316) eine Schleife (118, 316) umfasst, die sich durch den Schärfer (122, 314) hindurch und zwischen dem GWL (112, 312) und dem Freisetzungskatheter (102, 306) erstreckt.

14. System nach Anspruch 1, wobei der Freisetzungskatheter (102, 306) ferner einen Schärfer (122, 314) umfasst, der proximal zu dem distalen Ende (334) des Freisetzungskatheters (102, 306) positioniert ist, wobei der Schärfer (122, 314) dazu konfiguriert ist, das proximale Kopplungsmerkmal (118, 316) aufzunehmen, wobei das proximale Kopplungsmerkmal (118, 316) eine Schleife (118, 316) umfasst, die um die Außenfläche des GWL (112, 312) zwischenverbunden ist.

15. System nach Anspruch 1, wobei das proximale Kopplungsmerkmal (118, 316) eines von einer Durchzugseinrichtung, einem Fixierelement mit Gewinde, einer Schlinge, Schaufeln oder Arretierungen ist.

## Revendications

1. Système d'administration de capteur intracorporel (100, 300) comprenant :
un cathéter d'administration (102, 306) comprenant une lumière (308), le cathéter d'administration (102, 306) ayant une extrémité proximale (332) et une extrémité distale (334) ;
une lumière de fil-guide (GWL) (112, 312) configurée pour recevoir un fil-guide (114, 322), la GWL (112, 312) s'étendant à l'intérieur de la lumière (308) du cathéter d'administration (102, 306) et faisant saillie au-delà de l'extrémité distale (334) du cathéter d'administration (102, 306) ;
un capteur intracorporel (106) comprenant une extrémité proximale (328) et une extrémité distale (330), dans lequel l'extrémité proximale (328) du capteur intracorporel (106) est positionnée de manière distale par rapport à l'extrémité distale (334) du cathéter d'administration (102, 306) ;
un organe d'accouplement proximal (118, 316) accouplé à l'extrémité proximale (328) du capteur intracorporel (106), l'organe d'accouplement proximal (316) étant configuré pour accoupler de manière amovible le capteur intracorporel (106) au cathéter d'administration (102, 306) ; et
un organe d'accouplement distal (116, 324) accouplé à l'extrémité distale (330) du capteur intracorporel (106), **caractérisé en ce que** l'organe d'accouplement distal (324) soit en outre accouplé de manière amovible autour d'une surface externe de la GWL (112, 312) à une position qui est située de manière distale par rapport à l'extrémité distale (330) du capteur intracorporel (106).

2. Système selon la revendication 1, dans lequel l'organe d'accouplement distal (116, 324) comprend une boucle (116, 324) qui est entrelacée autour de la surface externe de la GWL (112, 312).

3. Système selon la revendication 2, dans lequel la boucle (116, 324) comprend :
une première partie de boucle (406) attachée au capteur intracorporel (106) au niveau d'un premier point d'attache (326a) ; et
une seconde partie de boucle (408) attachée au capteur intracorporel (106) au niveau d'un second point d'attache (326b), dans lequel les première et seconde parties de boucle (406, 408) de l'organe d'accouplement distal (116, 324) sont entrelacées pour former un premier ensemble de points de croisement (402a, 402b, 402c) le long d'un premier côté de la GWL (112, 312) et un second ensemble de points de croisement (404a, 404b, 404c) le long d'un second côté de la GWL (112, 312), dans lequel la première partie de boucle (406) se trouve à l'extérieur de la seconde partie de boucle (408) au niveau du premier ensemble de points de croisement (402a, 402b, 402c) et la seconde partie de boucle (408) se trouve à l'extérieur de la première partie de boucle (406) au niveau du second ensemble de points de croisement (404a, 404b, 404c).

4. Système selon la revendication 1, dans lequel l'organe d'accouplement distal (116, 324) comprend une boucle (116, 324) qui est torsadée ou enroulée autour de la surface externe de la GWL (112, 312).

5. Système selon la revendication 1, dans lequel l'organe d'accouplement proximal (118, 316) comprend une boucle (118, 316) qui est torsadée au moins une fois autour d'elle-même.

6. Système selon la revendication 1, dans lequel la GWL (112, 312) présente une longueur, dans lequel une première partie (386, 388) de la longueur présente une première rigidité, et une seconde partie (390) de la longueur présente une seconde rigidité qui est différente de la première rigidité.

7. Système selon la revendication 1, dans lequel l'organe d'accouplement distal (116, 324) comprend une boucle (116, 324) qui est enroulée autour de la surface externe de la GWL (112, 312) pour permettre à la boucle (116, 324), en réponse au désaccouplement de la GWL (112, 312) de l'organe d'accouplement distal (116, 324), de s'ouvrir latéralement sans torsion axiale, la boucle (116, 324) ouverte étant configurée pour venir en prise avec des parois d'un vaisseau pour assurer une stabilité en rotation du capteur intracorporel (106).

8. Système selon la revendication 1, comprenant en outre une gaine de pression (120, 600) configurée pour recouvrir de manière amovible une partie du cathéter d'administration (102, 306) et une partie de l'organe d'accouplement proximal (118, 316).

9. Système selon la revendication 1, dans lequel le capteur intracorporel (106) est un capteur de pression.

10. Système selon la revendication 1, dans lequel le cathéter d'administration (306) comprend en outre une seconde lumière (310) s'étendant parallèlement à la lumière (308).

11. Système selon la revendication 10, comprenant en outre une lumière de pression (313) positionnée en parallèle et maintenue solidaire de la seconde lumière (310) et de la GWL (312), dans lequel, éventuellement, la seconde lumière (310) est configurée pour faciliter des lectures de pression.

12. Système selon la revendication 10, dans lequel le système d'administration de capteur intracorporel (300) comprend en outre :
un câble de transmission de couple (321) s'étendant à travers la seconde lumière (310), le câble de transmission de couple (321) comprenant un organe d'accouplement proximal (323) configuré pour s'accoupler de manière amovible à l'extrémité proximale (328) du capteur intracorporel (106) ; ou
une tige de poussée (904) s'étendant à travers la seconde lumière (310), la tige de poussée (904) comprenant en outre un élément de coupe ou un câble de transmission de couple.

13. Système selon la revendication 1, dans lequel le cathéter d'administration (102, 306) comprend en outre un biseau (122, 314) pratiqué dans une lumière (308) et positionné de manière proximale par rapport à l'extrémité distale (334) du cathéter d'administration (102, 306), le biseau (122, 314) étant configuré pour recevoir l'organe d'accouplement proximal (118, 316), l'organe d'accouplement proximal (118, 316) comprenant une boucle (118,316) s'étendant à travers le biseau (122, 314) et entre la GWL (112,312) et le cathéter d'administration (102, 306).

14. Système selon la revendication 1, dans lequel le cathéter d'administration (102, 306) comprend en outre un biseau (122, 314) positionné de manière proximale par rapport à l'extrémité distale (334) du cathéter d'administration (102, 306), le biseau (122, 314) étant configuré pour recevoir l'organe d'accouplement proximal (118, 316), l'organe d'accouplement proximal (118, 316) comprenant une boucle (118, 316) entrelacée autour de la surface externe de la GWL (112, 312).

15. Système selon la revendication 1, dans lequel l'organe d'accouplement proximal (118, 316) est un élément parmi un fil dentaire, une fixation filetée, une anse, des palettes ou des crans de maintien.
